# EUROPEAN PATENT SPECIFICATION

(11) **EP 1 354 203 B1**
(45) Date of publication and mention of the grant of the patent: **16.05.2007**
(21) Application number: 01973369.0
(22) Date of filing: 21.09.2001
(51) Int. Cl.: G01N 33/574

(54) **BIOMARKERS OF TRANSITIONAL CELL CARCINOMA OF THE BLADDER**
BIOMARKIERUNGEN DES ÜBERGANGSZELLKARZINOMS DER BLASE
BIOMARQUEURS DU CARCINOME TRANSITIONNEL DE LA VESSIE

(30) Priority: 25.09.2000 US 235238 P
(43) Date of publication of application: 22.10.2003
(73) Proprietor: Eastern Virginia Medical School, Norfolk, VA 23507 (US)
(72) Inventor: VLAHOU, Antonia, Voula Attiki 16673 (GR); WRIGHT, George, L., Jr., Virginia Beach, VA 23455 (US)
(74) Representative: Maschio, Antonio
(86) International application number: PCT/US2001/029657
(87) International publication number: WO 2002/027329

(56) References cited:
- WO-A-00/19208
- WO-A-00/29987
- WO-A-01/36977
- WO-A-01/71360
- WO-A-98/59360
- US-A- 5 500 347
- RASMUSSEN H H ET AL: "TOWARDS A COMPREHENSIVE DATABASE OF PROTEINS FROM THE URINE OF PATIENTS WITH BLADDER CANCER" JOURNAL OF UROLOGY, BALTIMORE, MD, US, vol. 155, no. 6, June 1996 (1996-06), pages 2113-2119, XP001006003 ISSN: 0022-5347
- A. VLAHOU ET AL: "Development of a novel proteomic approach for the detection of transitional cell carcinome of the bladder in urine" AMERICAN JOURNAL OF PATHOLOGY, vol. 158, no. 4, 1 April 2001 (2001-04-01), pages 1491-1502, XP002201552

## Description

### BACKGROUND OF THE INVENTION

Bladder cancer is the second most common genitourinary malignancy accounting for approximately 5% of all newly diagnosed cancers in the United States (Klein *et al., Cancer,* 82 (2):49-354 (1998)). More than 90% are of the transitional cell carcinoma (TCC) histology (Stein *et al., J. Urol.,* 160:645-659 (1998)). At present, the most reliable way of diagnosis and surveillance of TCC is by cystoscopic examination and bladder biopsy for histologic confirmation. The invasive and labor-intensive nature of this procedure presents a challenge to develop better, less costly, and non-invasive diagnostic tools. Urine cytology has for many years been the 'gold standard' of the non-invasive approaches. It has high specificity and provides the advantage over biopsy of screening the entire urothelium (Klein *et al., Cancer,* 82 (2): 49-354 (1998); Stein *et al., J. Urol.,* 160:645-659 (1998)). However, its high false negative rate, particularly for low grade tumors, has limited its use as an adjunct to cystoscopy.

Many non-invasive molecular diagnostic tests have been developed based on an ever increasing knowledge about the molecular alterations associated with bladder cancer pathogenesis. The bladder tumor antigen (BTA) (Schamhart *et al., Eur. Urol.,* 34: 99-106 (1998)), the BTA stat (Sarosdy *et al*., *Urology,* 50:349-53 (1997)), the fibrinogen/fibrin degradation products (FDP) (Schmetter *et al*., *J. Urol.,* 158:801-805 (1997)) and the nuclear matrix protein-22 (NMP-22) (Soloway *et al*., *J. Urol.,* 156:363-367 (1996)) tests, have been approved by the FDA to be used in conjunction with cystoscopy. *See* Grossman *et al., Urol. Oncology,* 5:3-10 (2000) for review. Additional molecular assays currently being evaluated for their diagnostic/ prognostic utility (reviewed in 2, 3, 8, 9 ) are the Telomerase (Hoshi *et al., Urol. Onc.,* 5:25-30 (2000)), Immunocyt (Fradet *et al., Can J Urol.,* 1997, 4:400-S (1997)) and hyaluronic acid/ hyaluronidase (Pham *et al., Cancer Research,* 57:778-783 (1997); Lokeshwar *et al., Cancer research,* 57:773-777 (1997)) tests, microsatellite analysis (Steiner *et al., Nat. Med.,* 6:621-624. (1997)), as well as assays detecting blood group antigens (Golijanin *et al., Urology,* 46(2):173-177 (1995)), carcinoembryonic antigen (Liu *et al., J. Urol.,* 137:1258 (1987)), p53 and retinoblastoma proteins (Grossman *et al., Urol. Oncology,* 5:3-10 (2000)), E cadherin (Banks *et al., J. Clin. Pathol.,* 48:179-180 (1995); Protheroe *et al., British J. Cancer,* 80(1/2):273-8 (1999)), and various growth factors (Halachmi *et al., British J. Urology,* 82:647-654 (1998)).

WO-A-01/36977 (state of the art according to Art. 54 (3) EPC) discloses the use of mass spectrometry for detection of protein markers related with bladder carcinoma. WO-A-00/19208 and US-A-5,500,347 disclose methods for detecting bladder carcinoma based on the detection of specific protein markers. H. H. Rasmussen et al (1996), J. Urol. 155, 2113-2119 discloses a database of several protein markers found in patients with bladder carcinome and methods for detecting said markers in urine samples.

WO-A-98/59360 discloses the combination of retentate chromatography, protein chip arrays and desorption mass spectroscopy for the identification of a plurality of analytes present in a complex mixture. WO-A-00/29987 discloses a strategy based on combining MALDI-TOF mass spectrometry analysis with searching a protein sequence database for the identification of microorganisms present in a sample.

The effectiveness of any diagnostic test depends on its specificity and selectivity. That is, what is the relative ratio of true positive diagnoses, true negative diagnoses, false positive diagnoses and false negative diagnoses? Methods of increasing the percent of true positive and true negative diagnoses for any condition are desirable medical goals. In the case of bladder cancer, the present diagnostic tests are not completely satisfactory for the reasons described above.

Due to the molecular heterogeneity of TCC tumors, it is likely that there will be no single molecular assay that will replace cystoscopy. The identification and simultaneous analysis of a panel of biomarkers, representative of the various biological characteristics of the cancer, has greater potential for improving the early detection/diagnosis of TCC. Moreover, in an economy-conscious environment in which cost-effective medicine is an overriding concern, physicians treating cancer patients need convenient, efficient methods to rapidly diagnose bladder cancer and to evaluate responses to therapy. The present invention meets this and other goals.

### SUMMARY OF THE INVENTION

The present invention provides, diagnostic methods as defined in the appended claims based on the detection of novel protein markers that are differentially present in the samples of patients of transitional cell carcinoma of the bladder (TCC) and in the samples of control subjects. The measurement of these markers, alone or in combination, in patient samples provides information that diagnotician can correlate with a probable diagnosis of TCC or a negative diagnosis (e.g., normal or disease-free). All the markers are characterized by molecular weight. The markers can be resolved from other proteins in a sample by, *e.g.,* chromatographic separation coupled with mass spectrometry, or by traditional immunoassays. In preferred embodiments, the method of resolution involves Surface-Enhanced Laser Desorption/Ionization ("SELDI") mass spectrometry, in which the surface of the mass spectrometry probe comprises adsorbents that bind the markers.

A first set of markers is identified from urine samples, and is capable of binding to a cationic adsorbent and other adsorbents. These markers include Marker UBC-1: 3.353 kDa ± 105 Da, 3.432 kDa ± 122 Da, 3.470 kDa ± 32 Da; Marker UBC-2: 9.495 kDa ± 233 Da; Marker UBC-3: 44.6 kDa ± 1.9 kDa; Marker UBC-4: 100.120 kDa ± 4.3 kDa; Marker UBC-5: 133.190 kDa ± 3.9 kDa. These markers are detected as a single peak (except marker UBC-1 which is detected as two or three distinct peaks). Marker UBC-1 is also found in cell lysates of bladder barbotage and bind to a metal chelate adsorbent. Further analysis revealed that marker UBC-1 is a member of the defensin peptides.

A second set of markers is also identified from urine samples. These markers are also capable of binding to a cationic adsorbent and other adsorbents. These markers include Marker PC-1: about 4.950-5.150 kDa; Marker PC-2: about 5.710-6.000 kDa; Marker PC-3: about 6.758-7.750 kDa; Marker PC-4: about 15.000-16.000 kDa; Marker PC-5: about 37.500-40.000 kDa; Marker PC-6: about 79.500-82.000 kDa; and Marker PC-7: about 85.000-92.000 kDa. Except for marker PC-6, these markers are more frequently detected in TCC patients' samples than in the samples of control subjects (*e*.*g*., subjects with a negative TCC diagnosis). Marker PC-6 is more frequently detected in control samples and other non-TCC disease samples than in the sample of TCC samples.

While these markers were first identified from the urine sample, the sample from which they can be detected is not limited to a urine sample. These markers may be detectable in other types of samples, such as barbotage, blood, serum, tears, saliva, tissue, *etc.* For example, marker UBC-1 is also present in cell lysates of bladder barbotage. Moreover, although the first and second sets of markers were discovered using a cationic adsorbent (also a metal chelate adsorbent for marker UBC-1), the markers are capable of binding other types of adsorbents as described below. Accordingly, embodiments of the invention are not limited to the use of cationic adsorbents and metal chelate adsorbents.

While the absolute identity of these markers (except marker UBC-1) is not yet known, such knowledge is not necessary to measure them in a patient sample, because they are sufficiently characterized by, *e.g.,* mass and by affinity characteristics. It is noted that molecular weight and binding properties are characteristic properties of these markers and not limitations on means of detection or isolation. Furthermore, using the methods described herein or other methods known in the art, the absolute identity of the markers can be determined.

Accordingly, in one aspect the invention provides methods for aiding a TCC diagnosis, the method comprising (a) detecting at least one protein marker in a sample, wherein the protein marker is selected from Marker UBC-1: 3.353 kDa ± 105 Da, 3.432 kDa ± 122 Da, 3.470 kDa ± 32 Da; Marker PC-1: about 4.950-5.150 kDa; Marker PC-2: about 5.710-6.000 kDa; Marker PC-3: about 6.758-7.750 kDa; Marker PC-4: about 15.000-16.000 kDa; Marker PC-5: about 37.500-40.000 kDa; Marker PC-6: about 79.500-82.000 kDa; and Marker PC-7: about 85.000-92.000 kDa; and (b) correlating the detection of the marker or markers with a probable diagnosis of TCC.

In one embodiment, the correlation takes into account the amount of the marker or markers in the sample and/or the frequency of detection of the same marker or markers in a control.

In another embodiment, gas phase ion spectrometry is used for detecting the marker or markers. For example, laser desorption/ionization mass spectrometry can be used.

In another embodiment, laser desorption/ionization mass spectrometry used to detect markers comprises: (a) providing a substrate comprising an adsorbent attached thereto; (b) contacting the sample with the adsorbent; and (c) desorbing and ionizing the marker or markers from the substrate and detecting the desorbed/ionized marker or markers with the mass spectrometer. Any suitable adsorbents can be used to bind one or more markers. For example, the adsorbent on the substrate can be a cationic adsorbent, an antibody adsorbent, *etc.*

In another embodiment, an immunoassay can be used for detecting the marker or markers.

In another embodiment, methods further comprise (a) generating data on the sample with the mass spectrometer indicating intensity of signal for mass/charge ratios; (b) transforming the data into computer-readable form; and (c) operating a computer to execute an algorithm, wherein the algorithm determines closeness-of-fit between the computer-readable data and data indicating a diagnosis of TCC or a negative diagnosis.

### BRIEF DESCRIPTION OF THE DRAWINGS

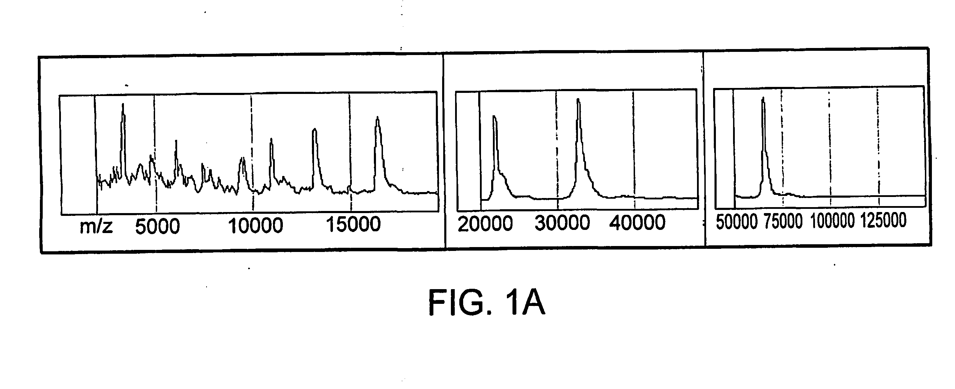

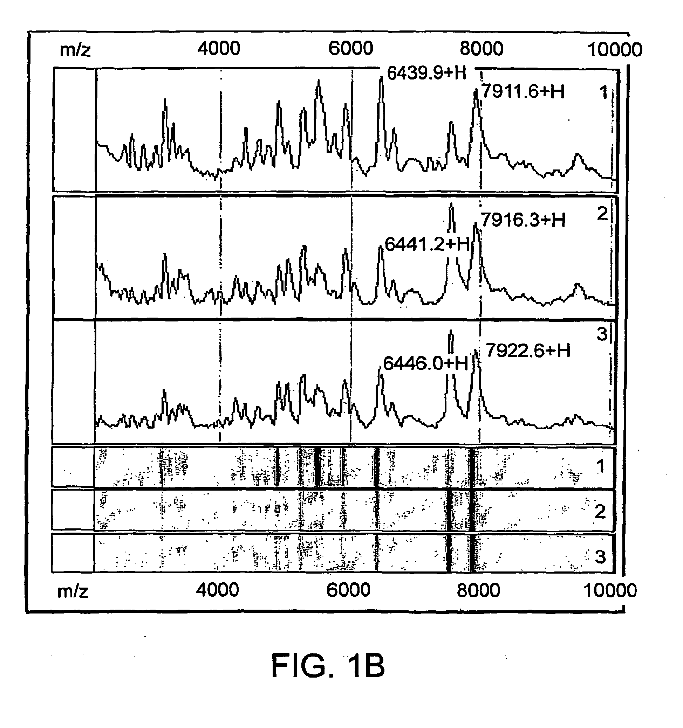

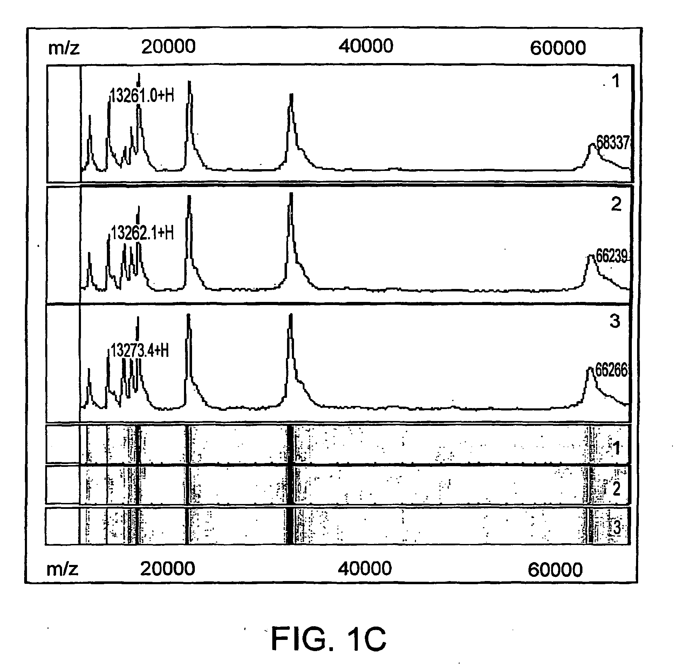

Figures 1a-c-illustrate protein mass spectra of urine samples. Figure 1a illustrates representative protein mass spectra of one urine sample processed on a strong anion exchange (SAX II) chip surface. Figures 1b and 1c illustrate reproducibility of protein detection using the SELDI-TOF-MS technology. 1-3: mass spectra (top) and respective gel views (bottom) of urine sample processed in triplicate. 1 and 2 were processed on the same day whereas 3 on a different day. Numbers correspond to the mass of the respective protein peaks. m/z: mass /charge (in Daltons).

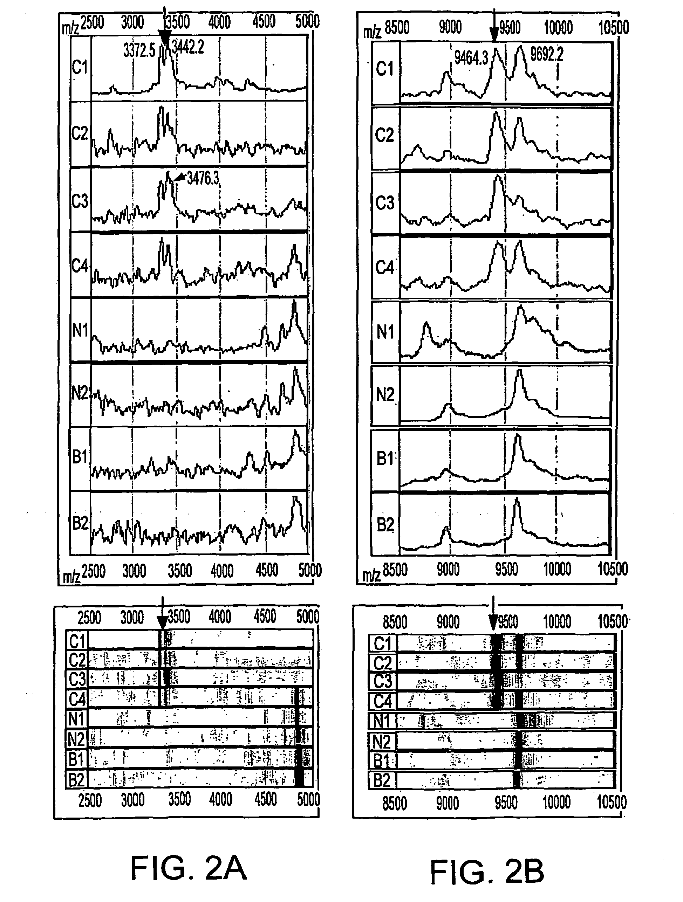

Figures 2a-d illustrates detection of 5 TCC-associated protein peaks in urine. Mass spectra (upper panel) and respective gel views (bottom panel) of urines from 4 different TCC patients (C1-C4), 2 Normals (N1-N2) and 2 patients with other urogenital diseases (B1-B2). The average molecular mass of the 5 proteins identified to be unique or overexpressed in the TCC specimens are: UBC-1: 3.352/3.432 kDa (a: arrow) and occasionally 3.47kDa (a: arrowhead); UBC-2. 9.495kDa (b: arrow); UBC-3: 44.647kDa (c: arrow); UBC-4: 100.120kDa, and UBC-5: 133.190 kDa (d: arrows). Numbers in the mass spectra represent the observed mass of the marker in that particular sample. M/z: mass/charge.

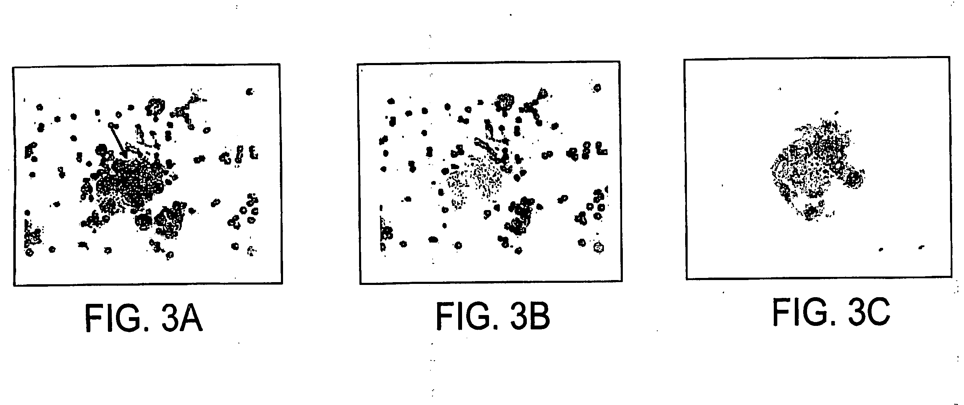

Figures 3a-c illustrate microdissection of pure populations of cancer cells from bladder washings. A: bladder washing cytospin. Arrow points to a cluster of cancer cells; B: same cytospin after microdissection of cancer cells; C: isolated cells. Stained with H. & E. at 40X.

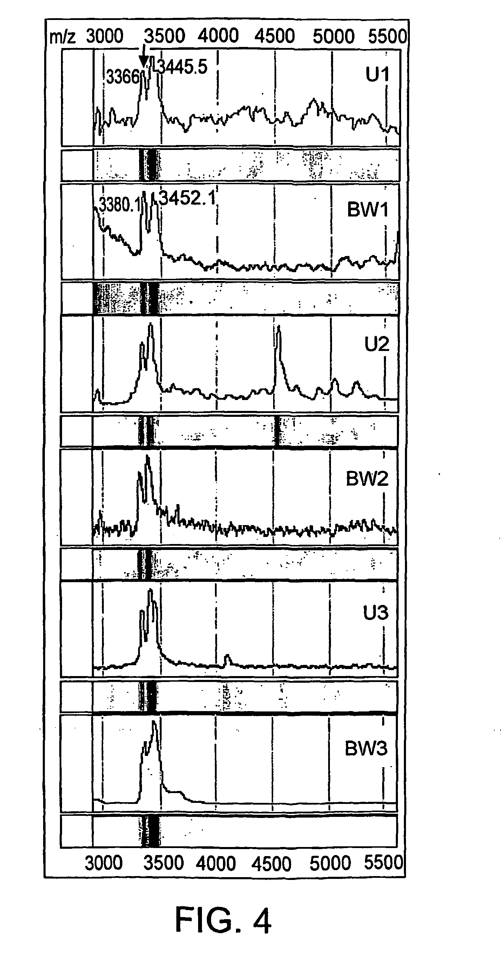

Figure 4 illustrates protein mass spectra and gel views of 3 sets of matched urines (U1-3) and cancer cells microdissected from bladder washings (BW1-BW3), showing the presence of the 3.35/3.43kDa protein (arrow) in the tumor cells and urine. M/z: mass/charge.

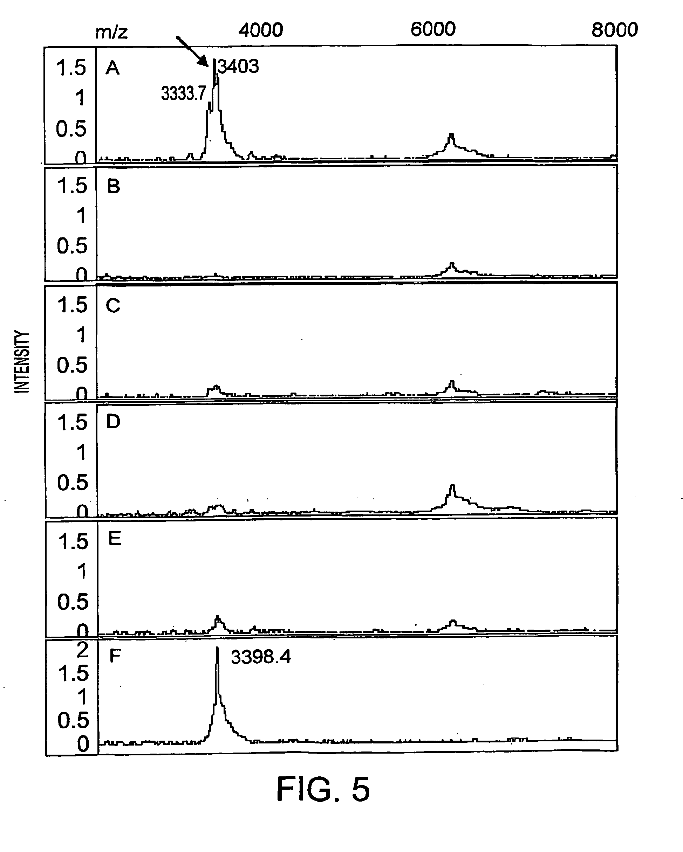

Figures 5A-F illustrate identification of the 3.3/3.4kDa (UBC-1) protein marker as defensin by SELDI immunoassay. A-D: A TCC urine, that by the direct binding SELDI assay contained the 3.3/3.4kDa marker, was incubated with either: A: defensin-a Ab; B: no Ab; C: an Ab reactive with prostate specific membrane antigen (PSMA); D: An irrelevant isotype matched control immunoglobulin; E: Normal urine that did not contam the 3.3/3.4kDa protein with defensin Ab. Note that the 3.3/3.4kDa protein was captured only in the sample containing this mass protein (panel A); F: pure α defensin peptide incubated with the α-defensin Ab. M/z:mass/charge.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton *et al., Dictionary of Microbiology and Molecular Biology* (2nd ed. 1994); *The Cambridge Dictionary of Science and Technology* (Walker ed., 1988); *The Glossary of Genetics,* 5th Ed., R. Rieger *et al.* (eds.), Springer Verlag (1991); and Hale & Marham, *The Harper Collins Dictionary of Biology* (1991). As used herein, the following terms have the meanings ascribed to them unless specified otherwise.

"Transitional cell carcinoma" or "TCC" refers to the carcinoma that develops in the very superficial cell lining of the bladder. The grade of the tumor reflects the degree of the aggressiveness of the disease and it is based on cellular and nuclear features, as defined by the pathologist. Grade I is considered the least, and grade III the most aggressive type. The stage reflects the degree of invasion and expansion of the tumor within the different layers of the bladder. In stage Ta, malignancy is confined to the most superficial layer (mucosa), in T1 invades the lamina propria, in T2 the muscularis mucosa, in T3 the perivesical fat and in T4 stage the tumor expands to adjacent organs. CIS (Carcinoma *in situ*) is a flat lesion confined to the mucosa.

"Marker" in the context of the present invention refers to a polypeptide (of a particular apparent molecular weight) which is differentially present in a sample taken from patients having TCC as compared to a comparable sample taken from control subjects (*e*.*g*., a person with a negative diagnosis or undetectable cancer, normal or healthy subject).

The phrase "differentially present" refers to differences in the quantity and/or the frequency of a marker present in a sample taken from patients having TCC as compared to a control subject. For example, a marker can be a polypeptide which is present at an elevated level or at a decreased level in samples of TCC patients compared to samples of control subjects. Alternatively, a marker can be a polypeptide which is detected at a higher frequency or at a lower frequency in samples of TCC patients compared to samples of control subjects. A marker can be differentially present in terms of quantity, frequency or both.

A polypeptide is differentially present between the two sets of samples if the frequency of detecting the polypeptide in the TCC patients' samples is statistically significantly higher or lower than in the control samples. For example, two sets of data can be compared using student's t-test, and P<0.05 can be considered statistically significant. In another example, a polypeptide is differentially, present between the two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

Alternatively or additionally, a polypeptide is differentially present between the two samples if the amount of the polypeptide in one sample is statistically significantly different from the amount of the polypeptide in the other sample. For example, a polypeptide is differentially present between the two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (*e.g.,* µg/ml) or a relative amount (*e.g.,* relative intensity of signals).

A "diagnostic amount" of a marker refers to an amount of a marker in a subject's sample that is consistent with a diagnosis of TCC (*e.g*., a healthy individual with a negative diagnosis of TCC). A diagnostic amount can be either in absolute amount (*e.g.,* µg/ml) or a relative amount (*e.g.,* relative intensity of signals).

A "control amount" of a marker can be any amount or a range of amount which is to be compared against a test amount of a marker. For example, a control amount of a marker can be the amount of a marker in a person without TCC. A control amount can be either in absolute amount (*e.g.,* µg/ml) or a relative amount (*e.g.,* relative intensity of signals).

"Probe" refers to a device that is removably insertable into a gas phase ion spectrometer and comprises a substrate having a surface for presenting a marker for detection. A probe can comprise a single substrate or a plurality of substrates. Terms such as ProteinChip®, ProteinChip® array, or chip are also used herein to refer to specific kinds of probes.

"Substrate" or "probe substrate" refers to a solid phase onto which an adsorbent can be provided (*e*.*g*., by attachment, deposition, etc.).

"Adsorbent" refers to any material capable of adsorbing a marker. The term "adsorbent" is used herein to refer both to a single material ("monoplex adsorbent") (*e*.*g*., a compound or functional group) to which the marker is exposed, and to a plurality of different materials ("multiplex adsorbent") to which the marker is exposed. The adsorbent materials in a multiplex adsorbent are referred to as "adsorbent species." For example, an addressable location on a probe substrate can comprise a multiplex adsorbent characterized by many different adsorbent species (*e.g.,* anion exchange materials, metal chelators, or antibodies), having different binding characteristics. Substrate material itself can also influence adsorbing properties of a marker.

"Adsorption" or "retention" refers to the detectable binding between an absorbent and a marker either before or after washing with an eluant (selectivity threshold modifier) or a washing solution.

"Eluant" or "washing solution" refers to an agent that can be used to mediate adsorption of a marker to an adsorbent. Eluants and washing solutions are also referred to as "selectivity threshold modifiers." Eluants and washing solutions can be used to wash and remove unbound materials from the probe substrate surface.

"Resolve," "resolution," or "resolution of marker" refers to the detection of at least one marker in a sample. Resolution includes the detection of a plurality of markers in a sample by separation and subsequent differential detection. Resolution does not require the complete separation of one or more markers from all other biomolecules in a mixture. Rather, any separation that allows the distinction between at least one marker and other biomolecules suffices.

"Gas phase ion spectrometer" refers to an apparatus that measures a parameter which can be translated into mass-to-charge ratios of ions formed when a sample is volatilized and ionized. Generally ions of interest bear a single charge, and mass-to-charge ratios are often simply referred to as mass. Gas phase ion spectrometers include, for example, mass spectrometers, ion mobility spectrometers, and total ion current measuring devices.

"Mass spectrometer" refers to a gas phase ion spectrometer that includes an inlet system, an ionization source, an ion optic assembly, a mass analyzer, and a detector.

"Laser desorption mass spectrometer" refers to a mass spectrometer which uses laser as means to desorb, volatilize, and ionize an analyte.

"Detect" refers to identifying the presence, absence or amount of the object to be detected.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, *e.g.,* by the addition of carbohydrate residues to form glycoproteins. The terms "polypeptide," "peptide" and "protein" include glycoproteins, as well as non-glycoproteins.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (*e.g*., as commonly used in an ELISA), biotin-streptavidin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantify the amount of bound detectable moiety in a sample. Quantitation of the signal is achieved by, *e.g.*, scintillation counting, densitometry, or flow cytometry.

"Antibody" refers to a polypeptide ligand substantially encoded by an immunoglobulin gene or immunoglobulin genes, or fragments thereof, which specifically binds and recognizes an epitope (*e*.*g*., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, *e.g.,* as intact immunoglobulins or as a number of well characterized fragments produced by digestion with various peptidases. This includes, *e.g.,* Fab' and F(ab)'₂ fragments. The term "antibody," as used herein, also includes antibody fragments either produced by the modification of whole antibodies or those synthesized *de novo* using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH₁, CH₂ and CH₃, but does not include the heavy chain variable region.

"Immunoassay" is an assay that uses an antibody to specifically bind an antigen (*e.g.,* a marker). The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to marker UBC-1 from specific species such as rat, mouse, or human can be selected to obtain only those polyclonal antibodies that are specifically immunoreactive with marker UBC-1 and not with other proteins, except for polymorphic variants and alleles of marker UBC-1. This selection may be achieved by subtracting out antibodies that cross-react with marker UBC-1 molecules from other species. A variety of immunoassay formats may be used to select antibodies specifically immunoreactive with a particular protein. For example, solid-phase ELISA immunoassays are routinely used to select antibodies specifically immunoreactive with a protein *(see, e.g.,* Harlow & Lane, *Antibodies, A Laboratory Manual* (1988), for a description of immunoassay formats and conditions that can be used to determine specific immunoreactivity). Typically a specific or selective reaction will be at least twice background signal or noise and more typically more than 10 to 100 times background.

"Energy absorbing molecule" or "EAM" refers to a molecule that absorbs energy from an ionization source in a mass spectrometer thereby aiding desorption of analyte, such as a marker, from a probe surface. Depending on the size and nature of the analyte, the energy absorbing molecule can be optionally used. Energy absorbing molecules used in MALDI are frequently referred to as "matrix." Cinnamic acid derivatives, sinapinic acid ("SPA"), cyano hydroxy cinnamic acid ("CHCA") and dihydroxybenzoic acid are frequently used as energy absorbing molecules in laser desorption of bioorganic molecules.

### DETAILED DESCRIPTION OF THE INVENTION

### I. INTRODUCTION

For many years, two-dimensional (2D) gel electrophoresis has been the principal tool for the separation and analysis of multiple proteins (O'Farrel *et al., J. Biol. Chem.,* 250:4007 (1975)). This methodology, which is able to resolve thousands of proteins in one experiment, provides the highest resolution in protein separation. However, it is labor intensive, requires large quantities of starting material, lacks interlab reproducibility and is not practical for clinical application. Although development of image analysis software for the comparison of 2D gel protein maps and automation of protein excision (Patterson, *Anal. Biochem.,* 221:1 (1994)) have facilitated the analysis of the separated proteins, most of the major technical difficulties of 2D gel electrophoresis remain.

Significant technological advances in protein chemistry in the last two decades have established mass spectrometry as an indispensable tool for protein study (Carr *et al., Anal. Chem.,* 63:2802 (1991); Carr *et al.,* "Overview of Peptide and Protein Analysis by Mass Spectrometry. Current Protocols in Molecular Biology," New York, John Wiley & Sons Inc., unit 10.21, pp. 10.21.1-10.21.27 (1998); Patterson, "Protein Identification and Characterization by Mass Spectrometry. Current Protocols in Molecular Biology," John Wiley & Sons Inc., unit 10.22, pp. 10.22.1-10.22.24 (1998)). Although the resolving power of 2D gels remains unchallenged, the high sensitivity, speed, and reproducibility of mass spectrometry have boosted its application in all aspects of protein analysis, including discovery, identification (*i.e.,* peptide mapping, sequencing), and structural characterization.

Analogous to the oligonucleotide microarray technologies that allow the study of gene expression profiles, Ciphergen Biosystems, Inc. has recently developed the ProteinChip^{®} technology coupled with SELDI-TOF-MS (surface-enhanced laser desorption/ionization time of flight mass spectrometry) to facilitate protein profiling of complex biologic mixtures (Hutchens *et al., Rapid Comm. Mass Spectrom,* 7:576-580 (1993); Kuwata *et al., Bioch. Bioph. Res. Comm.,* 245:764-773 (1998)). This technology utilizes patented chip arrays to capture individual proteins from complex mixtures which are subsequently resolved by mass spectrometry. This innovative technology has numerous advantages over 2D-PAGE: It is much faster, has a high throughput capability, requires orders of magnitude lower amounts of the protein sample, has a sensitivity for detecting proteins in the picomole to attamole range, can effectively resolve low mass proteins (2000-20000Da), and is directly applicable for clinical assay development.

The efficacy of the SELDI technology for discovery of prostate cancer protein markers in serum, seminal plasma and cell extracts, as well as the development of immunoassays for the detection of known prostate cancer markers has recently been demonstrated by our laboratory (Wright *et al., Prostate Cancer and Prostate Diseases,* 2:264-276 (1999); Paweletz *et al., Drug Development Research,* 49:34-42 (2000)). The present invention describes the novel TCC biomarkers detectable in urine as well as in other types of samples, and materials and methods for assessing these biomarkers for the diagnosis of TCC. Multiple protein changes were reproducibly found in the urine of TCC patients, including five novel urinary TCC biomarkers, and 7 protein cluster regions consisting of different number of proteins observed in the cancer versus the control groups. One of these urinary TCC-associated protein biomarkers was identified as belonging to the defensin family of peptides.

The TCC-associated protein biomarkers of the present invention can be used in various applications. For example, one or any combination of markers can be used to aid a TCC diagnosis. In another example, the markers can be used to screen for compounds that modulate the expression of the markers *in vitro* or *in vivo,* which compounds in turn may be useful in treating or preventing TCC in patients. In another example, markers can be used to monitor responses to certain treatments of TCC. In yet another example, the markers can be used in the heredity studies. For instance, certain markers may be genetically linked. This can be determined by, *e.g.*, analyzing samples from a population of TCC patients whose families have a history of TCC. The results can then be compared with data obtained from, *e.g.,* TCC patients whose families do not have a history of TCC. The markers that are genetically linked may be used as a tool to determine if a subject whose family has a history of TCC is pre-disposed to having TCC.

### II. CHARACTERIZATION OF MARKERS

Two sets of markers were identified from urine samples of TCC patients using gas phase ion spectrometry. Marker set 1 represents markers that were detected to have a single or defined mass value by gas phase ion spectrometry. Marker set 2 represents markers that were detected as protein clusters having a range of mass value by gas phase ion spectrometry. Each protein cluster marker of Marker Set 2 may represent a cluster of different proteins and/or a cluster of different states of the same protein.

### A. Marker Set 1

A first set of markers was found in urine samples of TCC patients. Optionally, urine samples were first fractionated by size exclusion chromatography followed by SELDI analysis on anion exchange chromatography. For example, the sample or an eluted fraction from size exclusion chromatography was applied to a substrate comprising a cationic adsorbent (*i*.*e*., SAX2 ProteinChip^{®}, Ciphergen Biosystems, Inc., Fremont, CA) and was tested by gas phase ion spectrometry to measure apparent molecular weights of markers retained on the adsorbent. These markers were present more frequently in TCC patients' urine samples compared to control samples. Table A below shows apparent molecular weights of each marker as measured by mass spectrometry.

**TABLE A**

| Marker | App. M.W. |
|---|---|
| UBC-1 | 3.353 kDa ± 105 Da, |
| | 3.432 kDa ± 122 Da, |
| | 3.470 kDa ± 32 Da |
| UBC-2 | 9.495 kDa ± 233 Da |
| UBC-3 | 44.6 kDa ± 1.9 kDa |
| UBC-4 | 100.120 kDa ± 4.3 kDa |
| UBC-5 | 133.190 kDa ± 3.9 kDa |

As shown in Table A, the apparent molecular weight of each marker is represented as a range. The variability range of mass for each marker (*e*.*g*., 233 Da for UBC-2) represents five times the standard deviation of mass measured by mass spectrometry from multiple samples *(see* the Example section). Since all of these markers bind to the cationic adsorbent, these markers are likely to have a net negative charge. Marker UBC-1 was also detected from cell lysates prepared from urine barbotage. Among these markers, marker UBC-1 is identified as human defensins a2 and 1. The identity of other markers is unknown. Other characteristics of these markers are further described in the example section below.

### B. Marker Set 2

A second set of markers was also found in urine samples of TCC patients using the sample procedure as for markers UBC-1 through UBC-5. Urine samples were first fractionated by size exclusion chromatography followed by SELDI analysis on anion exchange chip. Then, each fraction was applied to a substrate comprising an adsorbent with a cationic group (*i.e.,* SAX2 ProteinChip^{®}, Ciphergen Biosystems, Inc., Fremont, CA) and was tested by gas phase ion spectrometry to measure an apparent molecular weight of markers retained on the adsorbent. Except marker PC-6, these markers were present more frequently in TCC patients' urine samples compared to control samples. To the contrary, marker PC-6 was more frequently present in urine samples of healthy control group and non-TCC disease group compared to in urine samples from TCC disease group. Table B below shows apparent molecular weight of each marker.

**TABLE B**

| Marker | App. M.W. |
|---|---|
| PC-1 | about 4.950-5.150 kDa |
| PC-2 | about 5.710-6.000 kDa |
| PC-3 | about 6.758-7.750 kDa |
| PC-4 | about 15.000-16.000 kDa |
| PC-5 | about 37.500-40.000 kDa |
| PC-6 | about 79.500-82.000 kDa |
| PC-7 | about 85.000-92.000 kDa |

As shown in Table B, the apparent molecular weight for each marker (as measured by mass spectrometry) is referred to "about", because a molecular weight of a protein is typically resolved with confidence of about 0.5% variation by mass spectrometry. Therefore, "about" in the context of mass range of markers PC-1 through PC-7 refers to 0.5% variation of the values noted. For example, the apparent molecular weight range of marker PC-1 is 4.950 kDa ± 25 Da to 5.150 kDa ± 26 Da. For markers PC-2 through PC-7, the apparent molecular ranges of the markers are follows: Marker PC-2: 5.710 kDa ± 29 Da to 6.000 kDa ± 30 Da; Marker PC-3: 6.758 kDa ± 34 Da to 7.750 kDa ± 39 Da; Marker PC-4: 15.000 kDa ± 75 Da to 16.000 kDa ± 80 Da; Marker PC-5: 37.500 kDa ± 186 Da to 40.000 kDa ± 200 Da; Marker PC-6: 79.500 kDa ± 398 Da to 82.000 kDa ± 410 Da; and Marker PC-7: 85.000 ± 425 Da kDa to 92.000 kDa ± 460 Da.

Since all of these markers bind to the cationic adsorbent, these markers are likely to have a net negative charge. The identity of these markers is unknown. Other characteristics of these markers are further described in the example section.

While the markers were initially identified from a urine sample, the markers may be present in other types of samples (e.g., barbotage, blood, serum, saliva, tissue, etc.). Thus, samples from which the markers can be detected are not limited to a urine sample. Moreover, while the markers were initially identified using the techniques described above, the detection of the markers are not limited by these techniques and other techniques (e.g., ELISA immunoassays) can be used.

### III. DETECTION OF MARKERS

In another aspect, the invention provides methods for detecting markers which are differentially present in the samples of a TCC patient and a control (*e.g*., subjects in whom TCC is undetectable). The markers can be detected in a number of biological samples. The sample is preferably a biological fluid sample. Examples of a biological fluid sample useful in this invention include urine, bladder barbotage, blood, plasma, tears, saliva, tissue, *etc.* Because all of the markers are found in urine, urine is a preferred sample source for embodiments of the invention.

Any suitable methods can be used to detect one or more of the markers described herein. For example, gas phase ion spectrometry can be used. This technique includes, *e.g.,* laser desorption/ionization mass spectrometry. In some embodiments, the sample can be prepared prior to gas phase ion spectrometry, *e.g.,* pre-fractionation, two-dimensional gel chromatography, high performance liquid chromatography, *etc.* to assist detection of markers. Detection of markers can be achieved using methods other than gas phase ion spectrometry. For example, traditional immunoassays (e.g., ELISA) can be used to detect the markers in a sample. These detection methods are described in detail below.

### A. Detection by Gas Phase Ion Spectrometry

In a preferred embodiment, markers present in a sample are detected using gas phase ion spectrometry, and more preferably, using mass spectrometry. In one embodiment, matrix-assisted laser desorption/ionization ("MALDI") mass spectrometry can be used. In MALDI, the sample is typically quasi-purified to obtain a fraction that essentially consists of a marker or markers using protein separation methods such as two-dimensional gel electrophoresis or high performance liquid chromatography (HPLC).

In another embodiment, surface-enhanced laser desorption/ionization mass spectrometry ("SELDI") can be used. SELDI uses a substrate comprising adsorbents to capture markers, which can then be directly desorbed and ionized from the substrate surface during mass spectrometry. Since the substrate surface in SELDI captures markers, a sample need not be quasi-purified as in MALDI. However, depending on the complexity of a sample and the type of adsorbents used, it may be desirable to prepare a sample to reduce its complexity prior to SELDI analysis.

Various sample preparation methods to assist detection of markers in a sample and gas phase ion spectrometry methods are described in detail below.

### 1. Preparation of a Sample Prior to Gas Phase Ion Spectrometry

Optionally, one or combination of methods described below or other methods known in the art can be used to prepare a sample to further assist detection and characterization of markers in a sample. In some embodiments, a sample can be pre-fractionated to provide a less complex sample prior to gas phase ion spectrometry analysis. For example, a urine sample can be pre-fractionated according to size of proteins to reduce complexity of proteins in the sample. Moreover, pre-fractionation protocols can provide additional information regarding physical and chemical characteristics of markers. For example, if a sample was pre-fractionated using an anion-exchange spin column, and if a marker is eluted at a certain pH, this elution characteristic provides information regarding binding properties of the marker. In another example, a sample can be pre-fractionated by removing proteins or other molecules in the sample that are present in a high quantity or that may interfere with the detection of markers in a sample. Other suitable sample preparation protocols will be apparent to one of skill in the art, and they can also be applied in embodiments of the present invention.

### a) Size Exclusion Chromatography

In one embodiment, a sample can be pre-fractionated according to size of proteins in a sample using size exclusion chromatography. For a biological sample wherein the amount of sample available is small, preferably a size selection spin column is used. For example, K-30 spin column (Ciphergen Biosystems, Inc.) can be used. In general, the first fraction that is eluted from the column ("fraction 1") has the highest percentage of high molecular weight proteins; fraction 2 has a lower percentage of high molecular weight proteins; fraction 3 has even a lower percentage of high molecular weight proteins; fraction 4 has the lowest amount of large proteins; and so on. Each fraction can then be analyzed by gas phase ion spectrometry for the detection of markers.

### b) Separation of Biomolecules by Gel Electrophoresis

In another embodiment, biomolecules in a sample can be separated by high-resolution electrophoresis, *e.g*., one or two-dimensional gel electrophoresis. A fraction containing a marker can be isolated and further analyzed by gas phase ion spectrometry. Preferably, two-dimensional gel electrophoresis is used to generate two-dimensional array of spots of biomolecules, including one or more markers. *See, e.g.,* Jungblut and Thiede, *Mass Spectr. Rev.* 16:145-162 (1997).

The two-dimensional gel electrophoresis can be performed using methods known in the art. *See, e.g.,* Deutscher ed., *Methods In Enzymology* vol. 182. Typically, biomolecules in a sample are separated by, *e.g.*, isoelectric focusing, during which biomolecules in a sample are separated in a pH gradient until they reach a spot where their net charge is zero (*i*.*e*., isoelectric point). This first separation step results in one-dimensional array of biomolecules. The biomolecules in one dimensional array is further separated using a technique generally distinct from that used in the first separation step. For example, in the second dimension, biomolecules separated by isoelectric focusing are further separated using a polyacrylamide gel, such as polyacrylamide gel electrophoresis in the presence of sodium dodecyl sulfate (SDS-PAGE). SDS-PAGE gel allows further separation based on molecular mass of biomolecules. Typically, two-dimensional gel electrophoresis can separate chemically different biomolecules in the molecular mass range from 1000-200,000 Da within complex mixtures.

Biomolecules in the two-dimensional array can be detected using any suitable methods known in the art. For example, biomolecules in a gel can be labeled or stained (e.g., Coomassie Blue or silver staining). If gel electrophoresis generates spots that correspond to the molecular weight of one or more markers of the invention, the spot can be is further analyzed by gas phase ion spectrometry. For example, spots can be excised from the gel and analyzed by gas phase ion spectrometry. Alternatively, the gel containing biomolecules can be transferred to an inert membrane by applying an electric field. Then a spot on the membrane that approximately corresponds to the molecular weight of a marker can be analyzed by gas phase ion spectrometry. In gas phase ion spectrometry, the spots can be analyzed using any suitable techniques, such as MALDI or SELDI (e.g., using ProteinChip^{®} array) as described in detail below.

Prior to gas phase ion spectrometry analysis, it may be desirable to cleave biomolecules in the spot into smaller fragments using cleaving reagents, such as proteases (*e.g.*, trypin). The digestion ofbiomolecules into small fragments provides a mass fingerprint of the biomolecules in the spot, which can be used to determine the identity of markers if desired.

### c) High Performance Liquid Chromatography

In yet another embodiment, high performance liquid chromatography (HPLC) can be used to separate a mixture of biomolecules in a sample based on their different physical properties, such as polarity, charge and size. HPLC instruments typically consist of a reservoir of mobile phase, a pump, an injector, a separation column, and a detector. Biomolecules in a sample are separated by injecting an aliquot of the sample onto the column. Different biomolecules in the mixture pass through the column at different rates due to differences in their partitioning behavior between the mobile liquid phase and the stationary phase. A fraction that corresponds to the molecular weight and/or physical properties of one or more markers can be collected. The fraction can then be analyzed by gas phase ion spectrometry to detect markers. For example, the spots can be analyzed using either MALDI or SELDI (*e.g*., using ProteinChip^{®} array) as described in detail below.

### d) Modification of Marker Before Analysis

Optionally, a marker can be modified before analysis to improve its resolution or to determine its identity. For example, the markers may be subject to proteolytic digestion before analysis. Any protease can be used. Proteases, such as trypsin, that are likely to cleave the markers into a discrete number of fragments are particularly useful. The fragments that result from digestion function as a fingerprint for the markers, thereby enabling their detection indirectly. This is particularly useful where there are markers with similar molecular masses that might be confused for the marker in question. Also, proteolytic fragmentation is useful for high molecular weight markers because smaller markers are more easily resolved by mass spectrometry. In another example, biomolecules can be modified to improve detection resolution. For instance, neuraminidase can be used to remove terminal sialic acid residues from glycoproteins to improve binding to an anionic adsorbent (*e.g.,* cationic exchange ProteinChip^{®} arrays) and to improve detection resolution. In another example, the markers can be modified by the attachment of a tag of particular molecular weight that specifically bind to molecular markers, further distinguishing them. Optionally, after detecting such modified markers, the identity of the markers can be further determined by matching the physical and chemical characteristics of the modified markers in a protein database (*e.g.*, SWISS-PRO).

### 2. Contacting a Sample with a Substrate for Gas Phase Ion Spectrometry Analysis

A sample or a sample that is prepared as described above can be contacted with a substrate. A substrate can be a probe that is adapted for use with a gas phase ion spectrometer. Alternatively, a substrate can be a separate material that can be placed onto a probe that is adapted for use with a gas phase ion spectrometer. For example, a substrate can be a solid phase, such as a polymeric, paramagnetic, latex or glass bead comprising, *e.g.,* a functional group for binding markers. The substrate can then be positioned onto a probe. '

A probe can be in any suitable shape as long as it is adapted for use with a gas phase ion spectrometer (*e.g.*, removably insertable into a gas phase ion spectrometer). For example, the probe can be in the form of a strip, a plate, or a dish with a series of wells at predetermined addressable locations. The probe can also be shaped for use with inlet systems and detectors of a gas phase ion spectrometer. For example, the probe can be adapted for mounting in a horizontally and/or vertically translatable carriage that horizontally and/or vertically moves the probe to a successive position without requiring repositioning of the probe by hand.

The probe substrate can be made of any suitable material. For example, the probe substrate material can include, but is not limited to, insulating materials (*e.g.,* glass such as silicon oxide, plastic, ceramic), semi-conducting materials (*e.g.,* silicon wafers), or electrically conducting materials (*e.g.,* metals, such as nickel, brass, steel, aluminum, gold, or electrically conductive polymers), organic polymers, biopolymers, or any combinations thereof. The probe substrate material can also be solid or porous. Probes suitable for use in embodiments of the invention are described in, *e.g.*, U.S. Patent No. 5,617,060 (Hutchens and Yip) and WO 98/59360 (Hutchens and Yip).

### a) Analysis of Samples on an Inert Substrate

If complexity of a sample has been substantially reduced using the preparation methods described above, the sample can be contacted with any suitable substrate for gas phase ion spectrometry. For example, the substrate surface can be inert and need not comprise adsorbents for binding markers, since further separation of other biomolecules from markers is not necessary. In some embodiments, preferably a sample is prepared by two-dimensional gel electrophoresis or HPLC to obtain a fraction that contains markers prior to contacting the fraction with a substrate. Then the markers in the spot or fraction can be resolved using gas phase ion spectrometry (*e.g.,* traditional MALDI) without further fractionation, or using other known methods in the art.

Prior to gas phase ions spectrometry analysis, an energy absorbing molecule ("EAM") or a matrix material is typically applied to markers on the substrate surface. The energy absorbing molecules can assist absorption of energy from an energy source from a gas phase ion spectrometer, and can assist desorption of markers from the probe surface. Exemplary energy absorbing molecules include cinnamic acid derivatives, sinapinic acid ("SPA"), cyano hydroxy cinnamic acid ("CHCA") and dihydroxybenzoic acid. Other suitable energy absorbing molecules are known to those skilled in the art. *See, e.g.,* U.S. Patent 5,719,060 (Hutchens & Yip) for additional description of energy absorbing molecules.

The energy absorbing molecule and the sample containing markers can be contacted in any suitable manner. For example, an energy absorbing molecule is mixed with a sample containing markers, and the mixture is placed on the substrate surface, as in traditional MALDI process. In another example, an energy absorbing molecule can be placed on the substrate surface prior to contacting the substrate surface with a sample. In another example, a sample can be placed on the substrate surface prior to contacting the substrate surface with an energy absorbing molecule. Then the markers can be desorbed, ionized and detected as described in detail below.

### b) Analysis of Samples on a Substrate Surface Comprising Adsorbents

In some embodiments, complexity of a sample can be further reduced using a substrate that comprises adsorbents capable of binding one or more markers. Adsorbents need not be biospecific for markers (e.g., antibodies that specifically bind markers) as long as adsorbents have binding characteristics suitable for binding markers. For example, adsorbents can comprise a hydrophobic group, a hydrophilic group, a cationic group, an anionic group, a metal ion chelating group, lectin, heparin, or antibodies, or any combination thereof. Preferably, the adsorbent is cationic or comprises antibodies specific for markers.

Examples of various types of adsorbents are well-known in the art. For instance, adsorbents comprising a hydrophobic group include matrices having aliphatic hydrocarbons, *e.g.*, C₁-C₁₈ aliphatic hydrocarbons and matrices having aromatic hydrocarbon functional group such as phenyl groups. Adsorbents comprising a hydrophilic group include, ***e.g.*,** glass (*e.g.*, silicon oxide), or hydrophilic polymers such as polyethylene glycol, dextran, agarose, or cellulose. Adsorbents comprising a cationic group include, *e.g.*, matrices of secondary, tertiary or quaternary amines. Adsorbents comprising an anionic group include, *e.g.,* matrices of sulfate anions (SO₃⁻) and matrices of carboxylate anions (COO⁻) or phosphate anions (OPO₃⁻). Adsorbents comprising metal chelating groups include, *e.g.,* organic molecules that have one or more electron donor groups which form coordinate covalent bonds with metal ions, such as copper, nickel, cobalt, zinc, iron, and other metal ions such as aluminum and calcium. Adsorbents comprising an antibody include, *e.g.*, an antibody that specifically binds to any one of the markers provided herein. Probes with some of these adsorbents are also commercially available (*e.g.*, Normal Phase ProteinChip^{®}, SAX2 ProteinChip^{®}, IMAC3 ProteinChip^{®}, etc., all available from Ciphergen Biosystems, Inc. (Fremont, CA)). In preferred embodiments, adsorbents are substantially similar to or the same as the adsorbents which were initially used to enrich and identify the markers from a urine sample (e.g., SAX2 ProteinChip^{®}).

The probes can be produced using any suitable methods depending on the selection of substrate materials and/or adsorbents. For example, a metal surface can be coated with silicon oxide, titanium oxide or gold, and the coated surface can be derivatized with, *e.g.,* a bifunctional linker to bind and attach an adsorbent. For example, one end of a bifunctional linker can covalently bind with a functional group on the surface and the other end can be further derivatized with groups that function as an adsorbent. In another example, a porous silicon surface generated from crystalline silicon can be chemically modified to include adsorbents for binding markers. In another example, adsorbents can be formed directly on the substrate surface by *in situ* polymerizing a monomer solution which comprises, *e.g.*, substituted acrylamide monomers, substituted acrylate monomers, or derivatives thereof comprising a functional group of choice as an adsorbent. The polymerization of the monomer solution can provide hydrogel adsorbents with a greater capacity for binding biomolecules.

Adsorbents that bind the markers can be applied to the substrate in any suitable pattern (*e.g.*, continuous or discontinuous). For example, one or more adsorbents can be present on the substrate surface. If multiple types of adsorbents are used, the substrate surface can be coated such that one or more binding characteristics vary in one or two-dimensional gradient. If discontinuous, plural adsorbents can be on the substrate surface in predetermined addressable locations. The addressable locations can be arranged in any pattern, but are preferably in a regular pattern, such as lines, orthogonal arrays, or regular curves (*e.g.*, circles). For example, a probe can comprise discontinuous spots of adsorbents. Each addressable location may comprise the same or different adsorbent. The spots are "addressable" in that during mass spectrometry, an energy source, such as a laser, is directed to, or "addresses" each spot to desorb and ionize markers.

A sample is contacted with a substrate comprising an adsorbent in any suitable manner, *e.g.,* bathing, soaking, dipping, spraying, washing over, or pipetting, *etc.* Generally, a volume of sample containing from a few attomoles to 100 picomoles of marker in about 1 µl to 500 µl is sufficient for binding to the adsorbent. The sample can contact the probe substrate comprising an adsorbent for a period of time sufficient to allow the marker to bind to the adsorbent. Typically, the sample and the substrate comprising the adsorbent are contacted for a period of between about 30 seconds and about 12 hours, and preferably, between about 30 seconds and about 15 minutes. Typically, the sample is contacted to the probe substrate under ambient temperature and pressure conditions. For some samples, however, modified temperature (typically 4°C through 37°C) and pressure conditions can be desirable, which conditions are determinable by those skilled in the art.

After the substrate contacts the sample or sample solution, it is preferred that unbound materials on the substrate surface are washed out so that only the bound materials remain on the substrate surface. Washing a substrate surface can be accomplished by, *e.g.,* bathing, soaking, dipping, rinsing, spraying, or washing the substrate surface with an eluant. A microfluidics process is preferably used when an eluant is introduced to small spots of adsorbents on the probe. Typically, the eluant can be at a temperature of between 0°C and 100°C, preferably between 4°C and 37°C. In some embodiments, washing unbound materials from the probe surface may not be necessary if markers bound on the probe surface can be resolved by gas phase ion spectrometry without a wash.

Any suitable eluants (*e*.*g*., organic or aqueous) can be used to wash the substrate surface. Preferably, an aqueous solution is used. Exemplary aqueous solutions include a HEPES buffer, a Tris buffer, or a phosphate buffered saline, *etc.* To increase the wash stringency of the buffers, additives can be incorporated into the buffers. These include, but are limited to, ionic interaction modifier (both ionic strength and pH), water structure modifier, hydrophobic interaction modifier, chaotropic reagents, affinity interaction displacers. Specific examples of these additives can be found in, *e.g.*, PCT publication WO98/59360 (Hutchens and Yip). The selection of a particular eluant or eluant additives is dependent on other experimental conditions (e.g., types of adsorbents used or markers to be detected), and can be determined by those of skill in the art.

Prior to desorption and ionization of biomolecules including markers from the probe surface, an energy absorbing molecule ("TEAM") or a matrix material is typically applied to markers on the substrate surface. The types of EAM and the methods for applying EAM is discussed above, and will not repeated in this section.

### 3. Desorption/Ionization and Detection

Markers on the substrate surface can be desorbed and ionized using gas phase ion spectrometry. Any suitable gas phase ion spectrometers can be used as long as it allows markers on the substrate to be resolved. Preferably, gas phase ion spectrometers allow quantitation of markers.

In one embodiment, a gas phase ion spectrometer is a mass spectrometer. In a typical mass spectrometer, a substrate or a probe comprising markers on its surface is introduced into an inlet system of the mass spectrometer. The markers are then desorbed by a desorption source such as a laser, fast atom bombardment, high energy plasma, electrospray ionization, thermospray ionization, liquid secondary ion MS, field desorption, *etc.* The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of markers or other substances will typically involve detection of signal intensity. This, in turn, can reflect the quantity and character of markers bound to the substrate. Any of the components of a mass spectrometer (*e.g.,* a desorption source, a mass analyzer, a detector, *etc.)* can be combined with other suitable components described herein or others known in the art in embodiments of the invention.

Preferably, a laser desorption time-of-flight mass spectrometer is used in embodiments of the invention. In laser desorption mass spectrometry, a substrate or a probe comprising markers is introduced into an inlet system. The markers are desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of markers of specific mass to charge ratio.

In another embodiment, an ion mobility spectrometer can be used to detect markers. The principle of ion mobility spectrometry is based on different mobility of ions. Specifically, ions of a sample produced by ionization move at different rates, due to their difference in, *e.g.,* mass, charge, or shape, through a tube under the influence of an electric field. The ions (typically in the form of a current) are registered at the detector which can then be used to identify a marker or other substances in a sample. One advantage of ion mobility spectrometry is that it can operate at atmospheric pressure.

In yet another embodiment, a total ion current measuring device can be used to detect and characterize markers. This device can be used when the substrate has a only a single type of marker. When a single type of marker is on the substrate, the total current generated from the ionized marker reflects the quantity and other characteristics of the marker. The total ion current produced by the marker can then be compared to a control (e.g., a total ion current of a known compound). The quantity or other characteristics of the marker can then be determined.

### 4. Analysis of Data

Data generated by desorption and detection of markers can be analyzed using any suitable means. In one embodiment, data is analyzed with the use of a programmable digital computer. The computer program generally contains a readable medium that stores codes. Certain code can be devoted to memory that includes the location of each feature on a probe, the identity of the adsorbent at that feature and the elution conditions used to wash the adsorbent. The computer also contains code that receives as input, data on the strength of the signal at various molecular masses received from a particular addressable location on the probe. This data can indicate the number of markers detected, including the strength of the signal generated by each marker.

Data analysis can include the steps of determining signal strength (e.g., height of peaks) of a marker detected and removing "outerliers" (data deviating from a predetermined statistical distribution). The observed peaks can be normalized, a process whereby the height of each peak relative to some reference is calculated. For example, a reference can be background noise generated by instrument and chemicals (*e.g.*, energy absorbing molecule) which is set as zero in the scale. Then the signal strength detected for each marker or other biomolecules can be displayed in the form of relative intensities in the scale desired (*e.g*., 100). Alternatively, a standard (*e.g*., bovine serum albumin) may be admitted with the sample so that a peak from the standard can be used as a reference to calculate relative intensities of the signals observed for each marker or other markers detected.

The computer can transform the resulting data into various formats for displaying. In one format, referred to as "spectrum view or retentate map," a standard , spectral view can be displayed, wherein the view depicts the quantity of marker reaching the detector at each particular molecular weight. In another format, referred to as "peak map," only the peak height and mass information are retained from the spectrum view, yielding a cleaner image and enabling markers with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlaid to study subtle changes in relative peak heights. In yet another format, referred to as "difference map view," two or more spectra can be compared, conveniently highlighting unique markers and markers which are up- or downregulated between samples. Marker profiles (spectra) from any two samples may be compared visually. In yet another format, Spotfire Scatter Plot can be used, wherein markers that are detected are plotted as a dot in a plot, wherein one axis of the plot represents the apparent molecular of the markers detected and another axis represents the signal intensity of markers detected. For each sample, markers that are detected and the amount of markers present in the sample can be saved in a computer readable medium. This data can then be compared to a control (*e.g.,* a profile or quantity of markers detected in control, *e.g.,* subjects in whom TCC is undetectable).

### B. Detection by Immunoassay

In another embodiment, an immunoassay can be used to detect and analyze markers in a sample. This method comprises: (a) providing an antibody that specifically binds to a marker; (b) contacting a sample with the antibody; and (c) detecting the presence of a complex of the antibody bound to the marker in the sample.

To prepare an antibody that specifically binds to a marker, purified markers or their nucleic acid sequences can be used. Nucleic acid and amino acid sequences for markers can be obtained by further characterization of these markers. For example, each marker can be peptide mapped with a number of enzymes (*e*.*g*., trypsin, V8 protease, *etc.).* The molecular weights of digestion fragments from each marker can be used to search the databases, such as SWISS-PRO database, for sequences that will match the molecular weights of digestion fragments generated by various enzymes. Using this method, the nucleic acid and amino acid sequences of other markers can be identified if these markers are known proteins in the databases.

Alternatively, the proteins can be sequenced using protein ladder sequencing. Protein ladders can be generated by, for example, fragmenting the molecules and subjecting fragments to enzymatic digestion or other methods that sequentially remove a single amino acid from the end of the fragment. Methods of preparing protein ladders are described, for example, in International Publication WO 93/24834 (Chait *et al*.) and United States Patent 5,792,664 (Chait *et al.).* The ladder is then analyzed by mass spectrometry. The difference in the masses of the ladder fragments identify the amino acid removed from the end of the molecule.

If the markers are not known proteins in the databases, nucleic acid and amino acid sequences can be determined with knowledge of even a portion of the amino acid sequence of the marker. For example, degenerate probes can be made based on the N-terminal amino acid sequence of the marker. These probes can then be used to screen a genomic or cDNA library created from a sample from which a marker was initially detected. The positive clones can be identified, amplified, and their recombinant DNA sequences can be subcloned using techniques which are well known. *See, e.g., Current Protocols for Molecular Biology* (Ausubel *et al.,* Green Publishing Assoc. and Wiley-Interscience 1989) *and Molecular Cloning: A Laboratory Manual,* 2nd Ed. (Sambrook *et al.,* Cold Spring Harbor Laboratory, NY 1989).

Using the purified markers or their nucleic acid sequences, antibodies that specifically bind to a marker can be prepared using any suitable methods known in the art. *See, e.g.,* Coligan, *Current Protocols in Immunology* (1991); Harlow & Lane, *Antibodies: A Laboratory Manual* (1988); Goding, *Monoclonal Antibodies: Principles and Practice* (2d ed. 1986); and Kohler & Milstein, *Nature* 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (*see, e.g.,* Huse *et al., Science* 246:1275-1281 (1989); Ward *et al., Nature* 341:544-546 (1989)).

After the antibody is provided, a marker can be detected and/or quantified using any of suitable immunological binding assays known in the art *(see, e.g.,* U.S. Patent Nos. 4,366,241; 4,376,110; 4,517,288; and 4,837,168). Useful assays include, for example, an enzyme immune assay (EIA) such as enzyme-linked immunosorbent assay (ELISA), a radioimmune assay (RIA), a Western blot assay, or a slot blot assay. These methods are also described in, *e.g., Methods in Cell Biology: Antibodies in Cell Biology,* volume 37 (Asai, ed. 1993); *Basic and Clinical Immunology* (Stites & Terr, eds., 7th ed. 1991); and Harlow & *Lane, supra.*

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, *e.g.*, a microtiter plate, a stick, a bead, or a microbead. Antibodies can also be attached to a probe substrate or ProteinChip^{®} array and can be analyzed by gas phase ion spectrometry as described above. The sample is preferably a biological fluid sample taken from a subject. Examples of biological samples include urine, barbotage, blood, serum, plasma, tears, saliva, tissue, *etc.* In a preferred embodiment, the biological fluid comprises urine. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, *e.g.,* a second antibody which is labeled with a detectable label. Exemplary detectable labels include magnetic beads (*e.g.,* DYNABEADS™), fluorescent dyes, radiolabels, enzymes (*e.g.,* horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker is incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

Immunoassays can be used to determine the presence or absence of a marker in a sample as well as the quantity of a marker in a sample. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can be determined by comparing to a standard. A standard can be, *e.g.*, a known compound or another protein known to be present in a sample. As noted above, the test amount of marker need not be measured in absolute units, as long as the unit of measurement can be compared to a control.

The methods for detecting these markers in a sample have many applications. For example, one or more markers can be measured to aid TCC diagnosis or prognosis. In another example, the methods for detection of the markers can be used to monitor responses in a subject to cancer treatment. In another example, the methods for detecting markers can be used to assay for and to identify compounds that modulate expression of these markers *in vivo* or *in vitro.*

### IV. DIAGNOSIS OF TCC

In another aspect, the invention provides methods for aiding a TCC diagnosis using one or more markers in Marker Set 1 or Marker Set 2. These markers can be used alone, in combination with other markers in either set, or with entirely different markers (*e.g*., the bladder tumor antigen or NMP-22) in aiding TCC diagnosis. The markers in Marker Set 1 and Marker Set 2 are differentially present in samples of a TCC patient and a normal subject in whom TCC is undetectable. For example, all of the markers (except marker PC-6) are present at a higher frequency in TCC patients than in normal subjects. Therefore, detection of one or more of these markers in a person would provide useful information regarding the probability that the person has TCC.

Accordingly, embodiments of the invention include methods for aiding a TCC diagnosis, wherein the method comprises: (a) detecting at least one marker in a sample, wherein the marker is selected from Marker UBC-1: 3.353 kDa ± 105 Da, 3.432 kDa ± 122 Da, 3.470 kDa ± 32 Da; Marker UBC-2: 9.495 kDa ± 233 Da; Marker UBC-3: 44.6 kDa ± 1.9 kDa; Marker UBC-4: 100.120 kDa ± 4.3 kDa; Marker UBC-5: 133.190 kDa ± 3.9 kDa; Marker PC-1: about 4.950-5.150 kDa; Marker PC-2: about 5.710-6.000 kDa; Marker PC-3: about 6.758-7.750 kDa; Marker PC-4: about 15:000-16.000 kDa; Marker PC-5: about 37.500-40.000 kDa; Marker PC-6: about 79.500-82.000 kDa; and Marker PC-7: about 85.000-92.000 kDa; and (b) correlating the detection of the marker or markers with a probable diagnosis of TCC. The correlation may take into account the amount of the marker or markers in the sample compared to a control amount of the marker or markers (e.g., in normal subjects in whom TCC is undetectable). The correlation may take into account the presence or absence of the markers in a test sample and the frequency of detection of the same markers in a control. The correlation may take into account both of such factors to facilitate determination of whether a subject has a TCC or not.

Any suitable samples can be obtained from a subject to detect markers. Preferably, a sample is a urine sample from the subject. If desired, the sample can be prepared as described above to enhance detectability of the markers. For example, to increase the detectability of markers UBC-1 through UBC-5, a urine sample from the subject can be optionally fractionated by, *e.g.,* size exclusion chromatography. Sample preparations, such as pre-fractionation protocols, is optional and may not necessary to enhance detectability of markers depending on the methods of detection used. For example, sample preparation may be unnecessary if antibodies that specifically bind markers are used to detect the presence of markers in a sample.

Any suitable method can be used to detect a marker or markers in a sample. For example, gas phase ion spectrometry or a traditional immunoassay (*e.g.,* ELISA) can be used as described above. Using these methods, one or more markers can be detected. Preferably, a sample is tested for the presence of a plurality of markers. Detecting the presence of a plurality of markers, rather than a single marker alone, would provide more information for the diagnotician. Specifically, the detection of a plurality of markers in a sample would increase the percentage of true positive and true negative diagnoses and would decrease the percentage of false positive or false negative diagnoses.

The detection of the marker or markers is then correlated with a probable diagnosis of TCC. In some embodiments, the detection of the mere presence or absence of a marker, without quantifying the amount of marker, is useful and can be correlated with a probable diagnosis of TCC. For example, all of the markers (except marker UBC-6) are more frequently detected in TCC patients than in normal subjects. Thus, a mere detection of one or more of these markers in a subject being tested indicates that the subject has a higher probability of having a TCC.

In other embodiments, the detection of markers can involve quantifying the markers to correlate the detection of markers with a probable diagnosis of TCC. For example, some or all of the markers may be present at a higher quantity in urine samples of TCC patients than in urine samples of normal subjects. Thus, if the amount of the markers detected in a subject being tested is higher compared to a control amount, then the subject being tested has a higher probability of having a TCC.

An analysis of the data shows that detection of any one of markers UBC-1 through UBC-5 and PC-1 through PC-5 and PC-7 (or absence of marker PC-6) is not highly correlated with a positive diagnosis of TCC. However, the chance of a positive diagnosis increases significantly with the detection of more (*e*.*g*., two, three, four, and so on) of markers UBC-1 through UBC-5 and PC-1 through PC-5 and PC-7. Furthermore, the failure to detect any one of these markers UBC-1 through UBC-5 and PC-1 through PC-5 and PC-7 (or detection of marker UBC-6) also is not highly correlated with a negative diagnosis of TCC. However, the failure to detect many of these markers is highly correlated with a negative diagnosis of TCC.

When the markers are quantified, it can be compared to a control. A control can be, *e*.*g*., the average or median amount of marker present in comparable samples of normal subjects in whom TCC is undetectable. The control amount is measured under the same or substantially similar experimental conditions as in measuring the test amount. For example, if a test sample is obtained from a subject's urine sample and a marker is detected using a particular probe, then a control amount of the marker is preferably determined from a urine sample of a patient using the same probe. It is preferred that the control amount of marker is determined based upon a significant number of samples from normal subjects who do not have TCC so that it reflects variations of the marker amounts in that population.

Data generated by mass spectrometry can then be analyzed by a computer software. The software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal" and TCC and determines the closeness of fit between the two signals. Any suitable algorithm can be used. For example, an artificial intelligence program, such as fuzzy logic, cluster analysis of neural network (ANN) can be used. *See, e.g.,* Qureshi *et al., J. Urol.,* 163: 630-633 (2000); Snow, *et al., J. Urol.,* 152:1923 (1994). The software can also include code indicating which the test sample is closest to, thereby providing a probable diagnosis.

### EXAMPLES

The following examples are offered by way of illustration, not by way of limitation.

### I. SUBJECTS

Urine samples were collected over a period of several months from patients seen in the department of Urology, Eastern Virginia Medical School (EVMS). The urine samples were immediately aliquoted and stored at -80°C in the Tissue and Body Fluid Bank of the Virginia Prostate Center, until assayed. A total of 94 urine specimens were collected. The demographics of the TCC patient and control groups are provided in Table 1 as shown below.

| TABLE 1 | | | |
|---|---|---|---|
| | *TCC* | *Normal* | *Other* |
| # *of samples* | 30 | 34 | 30 |
| *age range* | 42-86 | 23-71 | 41-82 |
| *mean age* | 69.4 | 55 | 68.5 |

Healthy controls (n=34) included volunteers with no evidence of disease, and healthy individuals (*i.e.,* no history or evidence of urologic cancer) participating in the prostate cancer screening program at EVMS. TCC (n=30 patients) was histologically or cytologically confirmed at the time of specimen collection. In the case of recurrences none of the patients had received chemo- or immunotherapy within 3 months prior to specimen collection.

Grading was assessed using the World Health Organization (WHO) system. Tumor stage and grade of patients with TCC are shown in Table 2 below.

| TABLE 2 | | | |
|---|---|---|---|
| *Stage* | *# of samples* | *Grade* | *# of samples* |
| *Ta* | 14 | *I* | 4 |
| *T1* | 8 | *II* | 5 |
| *T2* | 5 | *III* | 21 |
| *T3* | 1 | | |
| *CIS* | 8 | | |

Four patients with Ta, 1 with T1 and 1 with T2 tumors had concomitant carcinoma *in situ* (CIS). Other urogenital diseases (n=30 patients) included clinical or pathologically confirmed prostatitis (n=6), prostatism (n=9), urinary tract infections (n=1), benign prostatic hyperplasia (BPH) (n=12), amyloidosis (n=1), inflammation of prostate and bladder (n=1), bladder outlet obstruction (n=1) and prostate cancer (n=1). One patient with benign prostate hyperplasia (BPH) and one with prostatism had concomitant prostatitis.

### II. STATISTICAL ANALYSIS

Sensitivity is defined as the ratio of the TCC patients that contained the biomarker to the total number of TCC patients included in the study. Specificity is defined as the ratio of the individuals that do not have the protein peak and do not have TCC, to the total number of individuals without TCC. Positive predictive value is defined as the probability that an individual with the biomarker has TCC. Negative predictive value is defined as the probability that an individual without the biomarker does not have TCC. Statistics were performed using the Chi Square test, after organizing the data in two-dimensional contingency tables and testing for independence of variables. Comparison of peak numbers between the various groups was performed utilizing student's t-test. In all cases, P<0.05 was considered statistically significant.

### III. PROTEIN BIOCHIP PRODUCTION

### A. SAX-2 ProteinChip^{®} (Strong anionic exchanger, cationic surface)

SAX-2 ProteinChip^{®} is a strong anion exchange array with a high capacity quaternary ammonium surface to bind anionic proteins. Anionic arrays bind proteins through electrostatic interaction of negatively charged amino acids such as aspartic acid and glutamic acid. Binding typically occurs at high pH with low salt, and binding decreases as pH decreases and salt concentration increases.

SAX-2 ProteinChip^{®} can be produced as follows. The surface of the metal substrate is conditioned and coated with a glass coating as described above. 3-(Methacryloylamino)propyl trimethylammonium chloride (15.0 wt%) and N,N'-methylenebisacrylamide (0.4 wt%) are photo-polymerized using (-)-riboflavin (0.01 wt%) as a photo-initiator and ammonium persulfate (0.2 wt%) as an accelerant. The monomer solution is deposited onto a rough etched, glass coated substrate (0.4 µL, twice) and is irradiated for 5 minutes with a near UV exposure system (Hg short arc lamp, 20 mW/cm² at 365 nm). The surface is washed with a solution of sodium chloride (1 M), and then the surface is washed twice with deionized water.

### B. IMAC3 ProtenChip^{®} (Immobilized Metal Affinity Capture, nitrilotriacetic acid on surface)

IMAC3 ProteinChip^{®} contains a surface for high-capacity metal binding and subsequent affinity capture of proteins with metal binding residues. Immobilized metal affinity capture arrays bind proteins and peptides which have affinity for metals; proteins with exposed histidine, tryptophan and/or cysteine typically bind to metals immobilized on these chip surfaces. Binding typically occurs under pH 6-8 and high salt, and binding decreases as the concentration of imidizole and glycine increase.

IMAC3 ProteinChip^{®} can be produced as follows. The surface of the metal substrate is conditioned and coated with a glass coating as described above. 5-Methacylamido-2-(N,N-biscarboxymethaylamino)pentanoic acid (7.5 wt%), Acryloyltri-(hydroxymethyl)methylamine (7.5 wt%) and N,N'-methylenebisacrylamide (0.4 wt%) are photo-polymerized using-(-)riboflavin (0.02 wt%) as a photo-initiator. The monomer solution is deposited onto a rough etched, glass coated substrate (0.4 mL, twice) and irradiated for 5 minutes with a near UV exposure system (Hg short arc lamp, 20 mW/cm2 at 365 nm). The surface is washed with a solution of sodium chloride (1 M) and then washed twice with deionized water.

IMAC3 ProteinChip^{®} with nickel can be activated with nickel metal ions as follows. The surface is treated with a solution of 100mM nickel sulfate to each spot and incubate on a high-frequency shaker at room temperature for 15 minutes. After removing the solution, the surface was rinsed with water. 5µL of 0.5M NaCl in PBS (or other binding buffer containing at least 0.5M NaCl) cam be added to each spot and incubate on shaker for 5 minutes. The spots are wipe dried.

### IV. PROTEIN BIOCHIP SELDI ANALYSIS OF URINE

### A. Urine Sample Preparation and SELDI Analysis

Urine samples were thawed and briefly centrifuged for the removal of cellular material. Protein concentration of the supernatants was estimated using the BCA kit (Pierce). Samples were diluted to a final protein concentration of 2mg/ml with binding buffer (20mM Tris pH 9, 0.4M NaCl, 0.1% Triton X 100) and subjected to protein size fractionation using a K30 micro-spin column (Ciphergen Biosystems, Inc). Following a 30min incubation in ice, diluted urines were applied to the spin columns and centrifuged for 3min at 720g. The ProteinChip^{®} SELDI analysis was performed similar to that described in an earlier report (Wright *et al., Prostate Cancer and Prostate Diseases,* 2:264-276 (1999)). Briefly, 5µl aliquots of the flow-through (fraction) and the unfractionated sample diluted in 20mM Tris pH 9.0, 0.1 % Triton-X 100, were directly applied onto different arrays of a SAX2 chip which consists of a strong anion exchanger chemistry. Following a brief wash with H₂O, 0.5µl of saturated matrix solution (alpha-cyano-4-hydroxycinnamic acid (CHCA) in 50% acetonitrile- 0.5% trifluoroacetic acid) was applied on the array and allowed to air dry. The chips were then placed in the PBS-I mass reader, where nanosecond laser pulses are generated from a nitrogen laser (337nm). Spectra were generated using an average of 60 laser shots at each of the following laser intensities (L): 15 (filter in), 30 (filter in) and 55 (filter out). For the calculation of protein peak numbers resolved at low laser intensities, spectra collected at L15 and L30 were combined utilizing the SELDI software (0.5% variation). External calibration was performed utilizing Bovine Insulin (5733.6Da), Bovine Cytochrome C (12230.9Da), and Bovine Serum Albumin (66410Da) as standards (Ciphergen Biosystems).

Ninety-four urine samples were assayed by SELDI mass spectrometry. Processing on a strong anion exchanger chip surface resolved up to 70 protein peaks. Figure la is a representative protein spectrum showing the protein masses between 2,000-150,00ODa of a single urine specimen. Generation of spectra was performed at laser intensities 15-30 and 55, so as to better resolve low and high molecular mass proteins, respectively. As shown, the SELDI technology was particularly effective in resolving the low molecular weight (<10kDa) proteins and polypeptides. Interestingly, urines from TCC patients appeared to contain higher numbers of protein peaks. Collection of data at laser intensities 15 and 30, generated an average of 33 protein peaks from the TCC urines versus average of 21 and 22 for the normal and other urogenital diseases, respectively (P<0.001). Similarly, at higher laser intensities (*i.e.,* 55-filter out), TCC samples had an average of 34 protein peaks, versus 27 and 20 in the normal and other urogenital diseases groups (P<0.001 for the normals and P=0.008 for the other diseases).

All samples were processed in either duplicate or triplicate to confirm reproducibility in resolving the urinary proteins. Figures 1b-c show that reproducibility was quite acceptable. The mean, standard deviation (SD) and coefficient of variation (CV) were determined for 4 prominent peaks, designated as proteins 1-4. The intra-assay reproducibility, *i*.*e*., the mean mass, SD (%CV) for protein 1 was 6440.6±0.92Da (0.014%), for protein 2, 7914±3.32Da (0.042%), for protein 3, 13262+0.78Da (0.006%) and for protein 4, 66288±69.3Da (0.1%) (Figs. 1b-c, spectra 1 and 2). The inter-assay reproducibility was determined to be 6443.3±3.85Da (0.06%) for protein 1, 7918.3±6.12Da (0.08%) for protein 2, 13267±8.42Da (0.06%) for protein 3 and 66277±15.56Da (0.023%) for protein 4 (Figs. 1b-c, spectra 1 and 2 versus 3).

### B. Marker Set 1: UBC-1 through UBC-5

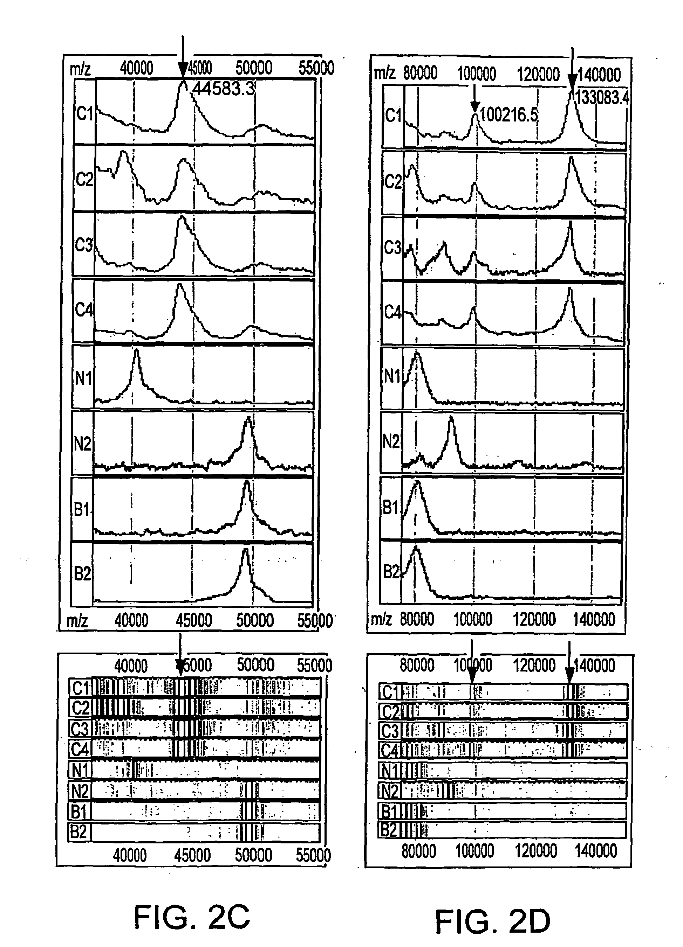

Analysis of urine specimens from patients with TCC, patients with other diseases of the urogenital tract and normal individuals, revealed that 5 prominent protein peaks were preferentially expressed in TCC. Representative mass spectra and gel views of these proteins are shown in Figure 2. One of the proteins was observed as a doublet or occasionally as a triplet protein peak (Fig 2a) having an average mass of 3.353 (SD:21Da), 3.432 (SD:24.4Da) and 3.470kDa (SD:6.32Da), respectively. This protein will be referred to as marker UBC-1. The average SELDI mass associated with the other 4 TCC-associated proteins are UBC-2: 9.495kDa (SD: 46.5Da); UBC-3: 44.6kDa (SD:372.8Da); UBC-4: 100.120kDa (SD: 866.8); and UBC-5: 133.190kDa (SD:772.9Da) (Figs 2b-d).

Table 3 shown below illustrates statistical analysis of detection of the five TCC associated UCB markers in the study and control groups.

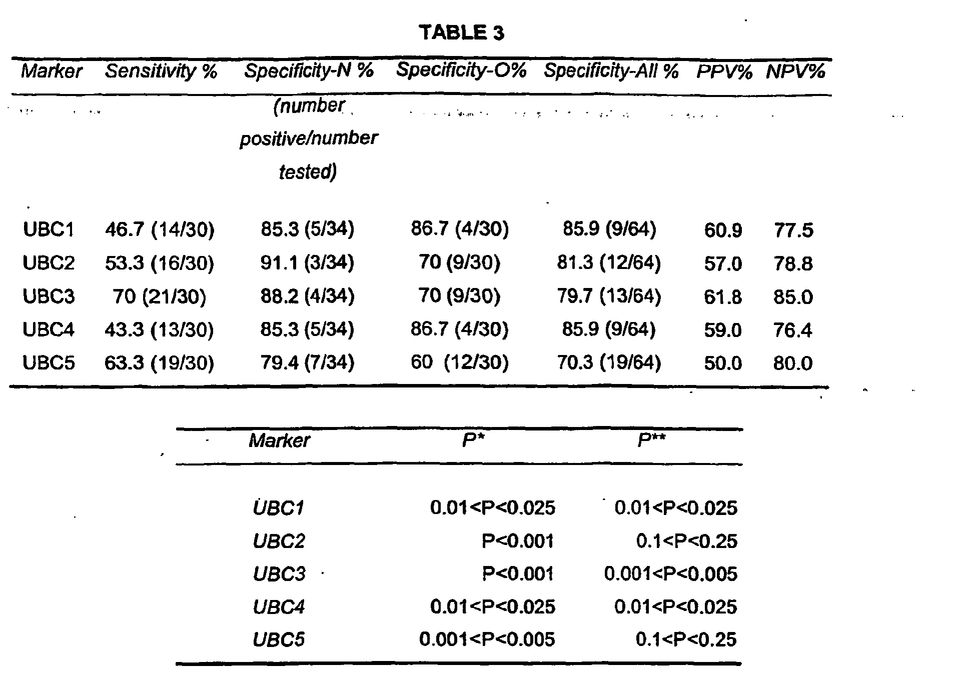

Of the TCC patient urines evaluated, 46.7% (14/30) were positive for UBC-1; 53.3% (16/30) for UBC-2; 70% (21/30) for UBC-3; 43.3% (13/30) for UBC-4; and 63.3% (19/30) for UBC-5 (Table 3).

The expression of the markers with regard to stage and grade of TCC is shown in Table 4 below.

**TABLE 4**

| | | *number of positive*/*total number tested (%)* | | | | |
|---|---|---|---|---|---|---|
| *Marker* | *Grade I-II* | *Grade III* | *Stage Ta* | *Stage T1* | *Stage T2-T3* | *CIS* |
| *UBC1* | 4/9 (44.4) | 10/21 (47.6) | 5/14 (35.7) | 6/8(75) | 3/6 (50) | 4/8 (50) |
| *UBC2* | 4/9 (44.4) | 12/21 (57.1) | 6/14 (42.8) | 4/8 (50) | 5/6 (83.3) | 4/8 (50) |
| *UBC3* | 4/9 (44.4) | 16/21 (80.9) | 7/14 (50) | 7/8 (87.5) | 5/6 (83.3) | 4/8 (50) |
| *UBC4* | 2/9 (22.2) | 11/21 (52.4) | 6/14 (42.8) | 3/8 (37.5) | 3/6 (50) | 3/8 (37.5) |
| *UBC5* | 2/9 (22.2) | 15/21 (71.4) | 8/14 (57) | 7/8 (87.5) | 3/6 (50) | 4/8 (50) |

Although the TCC patient population was unselected with regard to stage and grade, it is notable that the majority of the malignancies were superficial cancers (stages Ta/T1; grade III) (Table 2). Frequency of almost all markers was observed to increase with progression from low grade (I-II) to high grade (III) and low stage (Ta) to higher stage (T1-3) carcinomas.

The percent positive samples for the 5 biomarkers in the normal population were 14.7 (5/34) for UBC-1; 8.9 (3/34) for UBC-2; 11.8 (4/34) for UBC-3; 14.7 (5/34) for UBC-4; and 20.6 (7/34) for UBC-5, corresponding to a specificity of 85.3%, 91.1%, 88.2%, 85.3% and 79.4%, respectively (Table 3). The frequency of the markers in this control group is significantly different than their frequency in the TCC urines (Table 3).

Biomarkers UBC-1 and UBC-4 were found to be present in urine specimens from patients with other urogenital diseases at a frequency (4/30 or 13.3%) nearly equal to the normal group. Markers UBC-2, -4 and -5 however, were found at relatively higher frequencies: 30% (9/30) for UBC-2 and UBC-4, and 40% (12/30) for UBC-5. The difference in the frequency of the markers between this control (*i.e.*, other diseases) and the TCC cancer group remains statistically significant for markers UBC-1, -3, and -4, but was not significant for markers UBC-2 and UBC-5 (Table 3).

Based on these results, the overall specificity of the individual markers for TCC detection range from 70.3-85.9% (Table 3). Similarly, the negative predictive values (NPV) varied from 76.4-85%, and the positive predictive values (PPV) from 50-61.8% (Table 3).

### C. Marker Set 2: PC-1 through PC-7

In addition to the detection of differences in the frequency of individual protein peaks between the TCC and the control groups, regional differences in the mass spectra were also observed. Statistical analysis of detection of protein clusters with differential expression in the study and control groups is shown in Table 5 below.

**TABLE 5**

| | | *Number of positive*/*total number positive* | | | | |
|---|---|---|---|---|---|---|
| *Marker* | *Mass range (kD)* | *TCC* | *Normal* | *Other* | *P** | *P*** |
| *PC-1* | *4.950-5.150* | 17/30 (56.7) | 9/34 (26.5) | 2/30 (6.7) | 0.025<P<0.05 | P<0.001 |
| PC-2 | *5.710-6,000* | 15130 (50) | 4/34 (11.8) | 6/30 (20) | 0.001 <P<0.005 | 0.025<P<0.05 |
| *PC-3* | *6.758-7.750* | 20/30 (66.7) | 4/34 (11.8) | 11/30 (36.7) | P<0.001 | 0.025<P<0.05 |
| *PC-4* | *15.000-16.000* | 19/30 (63.3) | 8/34 (23.5) | 7/30 (23.3) | 0.001<P<0.005 | 0.001<P<0.005 |
| *PC-5* | *37.500-40.000* | 20/30 (66.7) | 7/34 (20.6) | 16/30 (53.3) | P<0.001 | 0.75<P<0.9 |
| *PC-6* | *79.500-82.000* | 10/30 (33.3) | 22/34 (64.7) | 24/30 (80) | 0.001<P<0.005 | P<0.001 |
| PC-7 | *85.000-92.000* | 15/30 (50) | 5/34 (14.7) | 5/30 (16.7) | 0.005<P<0.01 | 0.01<P<0.025 |

Table 5 shows the number and percent positive, and p values for the 7 protein cluster regions that demonstrated differences between the TCC group and control groups. The protein pattern displayed by 5 of these clusters, including 4,950-5,150Da, 5,710-6,000Da, 6,758-7,750Da, 15,000-16,00ODa, and 85,000-92,000Da, was found to be significantly different in urines from TCC patients than the patterns found in the healthy and other disease controls. The only exception was the 37,500-40,000 region which was found not to be statistically (0.75<p<0.9) different between the TCC and the other diseases group. Interestingly, a protein cluster with masses ranging from 79.5-82.0 kDa was found in 64.7% of the healthy control group and in 80% of urines from the non-TCC disease group but in only 33% of the TCC group. The difference in frequency of this cluster between the control and the TCC groups was statistically significant.

Table 6 below shows the distribution of the protein clusters with TCC stage and grade.

**TABLE 6**

| | | | *Number of positive*/*total number tested positive* | | | | |
|---|---|---|---|---|---|---|---|
| *Marker* | *Mass range (kD)* | *Grade I-II* | *Grade III* | *Stage Ta* | *Stage T1* | *Stage T2-T3* | *CIS* (%) |
| *PC-1* | *4.950-5.150* | 219 (22.2) | 15/21 (71.4) | 5/14 (35.7) | 6/8 (75) | 5/6 (83.3) | 4/8 (50) |
| *PC-2* | *5.710-6.000* | 3/9 (33.3) | 12/21 (57.1) | 6/14 (42.9) | 4/8 (50) | 4/6 (66.7) | 2/8 (25) |
| *PC-3* | *6.758-7.750* | 5/9(55.5) | 15/21 (71.4) | 9/14 (64.3) | 5/8 (62.5) | 5/6 (83.3) | 5/8 (62.5) |
| *PC-4* | *15.000-* | 5/9 (55.5) | 14/21 (66.7) | 8/14 (57.1) | 7/8 (87.5) | 3/6 (50) | 5/8 (62.5) |
| | *16.000* | | | | | | |
| *PC-5* | *37.500-* | 6/9 (66.7) | 14/21 (66.7) | 9/14 (64.3) | 5/8 (62.5) | 4/6 (66.7) | 5/8 (62.5) |
| | *40.000* | | | | | | |
| *PC-6* | *79.500-* | 3/9 (33.3) | 7/21 (33.3) | 3/14 (21.4) | 4/8 (50) | 3/6 (50) | 2/8 (25) |
| | *82.000* | | *.* | | | | |
| PC-7 | *85.000-* | 3/9 (33.3) | 12/21 (57.1) | 7/14 (50) | 4/8 (50) | 3/6 (50) | 2/8 (25) |
| | *92.000* | | | | | | |

Similar to the UBC1-5 markers, the frequency of most of the clusters was observed to increase with progression from grades I-II to grade III and stage Ta to stages T1-3 carcinomas.

Not wishing to be bound by a theory, since these clusters, with the exception of the one between 79.5-82.0 kDa, were observed preferentially in the TCC group, they may be considered as a reflection of increased protein excretion in urine of bladder cancer patients detected herein and reported earlier (Protheroe *et al., British J. Cancer,* 80(1/2):273-8 (1999); Hemmingsen *et al., J. Urol.,* 152:1923 (1994)) and attributed either to leakage of serum proteins from the tumor neovasculature, or to increased turnover of bladder cancer cells (Protheroe *et al., British J. Cancer,* 80(1/2):273-8 (1999)).

### V. PROTEIN BIOCHIP SELDI ANALYSIS OF BLADDER BARBOTAGE

### A. Bladder Barbotage Sample Preparation and SELDI Analysis

Bladder washings were centrifuged at 1,500rpm for 5 min for the collection of cellular material. Supernatants were discarded with the exception of 1-2 mis which was used for resuspending the cell pellet Cytospin preparations of 50-100µl of the resuspended cell pellet were then made, the slides immediately placed in 100% EtOH, and stained with hematoxylin and eosin. The stained slides were examined by a pathologist (S.N.) to identify the cancer cells, and the individual cancer cells or clusters were procured using the Pixcell 100 Laser Capture Microdissection Microscope (Arcturus Engineering, Mountain View, CA), as previously described (Wright *et al.* (1999), *supra;* Emmert-Buck *et al., J. Immun. Meth.,* 141:149-155 (1991)).

Protein extracts were prepared from 500-1000 microdissected cells by resuspending the cells in 3-5 µl of 20mM HEPES containing 0.1% NP-40, vortexing for 5 min, and then centrifugation at 14,000 rpm for 1 min. The entire lysate was applied onto a nickel IMAC3 (Immobilized Metal Affinity) ProteinChip^{®} array, and incubated for 1 hr. The chips were washed with 20mM Tris pH 7.5, 0.1% Triton X-100, 0.5M NaCl (5 times), and HPLC-H₂O (5 times). Mass analysis was performed as described for urine, using either CHCA or sinapinic acid (SPA) as the energy absorbing molecules.

### B. Detection of Marker UBC-1

A total of 6 matched (*i*.*e*., from the same TCC patient) bladder washing and urine specimen sets were analyzed. Bladder cancer cells from all 6 patients expressed the 3.3/3.4kDa protein (the UBC-1 marker) which was also present in 4/6 matched urines. Figure 4 shows 3 of the matched sets that were positive for the marker in both cell lysate and urine. It is notable, that the doublet peak pattern for this protein found in urine is maintained in the spectra of the cell lysates. Bladder epithelial cells from 2 different bladder barbotage specimens, characterized by the pathologist as benign, were also found to contain the 3.3/3.4kDa protein (data not shown).

In contrast to the UBC-1 protein marker, the 9.5 (UBC-2), 44 (UBC-3), 100 (UBC-4) and 133 (UBC-5) kDa urinary proteins were not detected in the bladder cell lysates. Not wishing to be bound by a theory, markers UBC-2 through UBC-5 may be extracellular proteins, or alternatively, proteolytic fragments of intracellular proteins, since they were not detected in cancer cells procured from cytology specimens. If desired, the identification of these proteins can be achieved by, *e.g.*, tryptic peptide mapping (Patterson, *Anal. Biochem.,* 221:1 (1994)) and amino acid sequencing (Patterson, SD, "Protein Identification and Characterization by Mass Spectrometry. Current Protocols in Molecular Biology," John Wiley & Sons Inc., unit 10.22, pp. 10.22.1-10.22.24 (1998)).

### C. Identification of Marker UBC-1 as defesin family

Searching through protein databases (SWISS-PRO; www.expasy.ch/tools/tagident.html) for proteins with similar molecular weight to the 5 TCC-associated markers, suggested that the doublet 3.3/3.4kDa marker correspond to human defensins α 2 and 1 (Celis *et al., Electrophoresis,* 20(2):300-9 (1999)) with reported molecular mass of 3.38 and 3.45 kDa, respectively. To test this hypothesis, a SELDI-based immunoassay was performed utilizing a commercially available antibody against human defensins 1, 2 and 3.

The SELDI immunoassay was performed similar to that described in a previous report (Wright *et al.* (1999), *supra).* Briefly, the arrays of a preactivated chip (PS 1 ProteinChip^{®}, Ciphergen Biosystems, Inc., Fremont, CA), were coated with 4µl Protein G (0.5mg/ml in 50mM sodium bicarbonate pH.8, Sigma) for 2-4hrs at room temperature with shaking. Residual active sites were subsequently blocked with 1 M ethanolamine (30min, R.T), followed by sequential washes in 15ml conical tubes with PBS-0.5% Triton X (x3) and PBS (x4). 2µl of defensins 1-3 (HNP1, 2 and 3) monoclonal antibody (Ab) (IgG1, 0.2mg/ml, Serotec), PSMA 7E11C5.3 Ab (IgG1, 0.2mg/ml, kindly provided by Cytogen Corporation, Princeton, NJ) or mouse IgG1 (30µg/ml) were applied on the chip and allowed to bind at 4°C, overnight (o/n) with shaking. Unbound Abs were removed by sequential washes in 15ml conical tubes with PBS -0.5% TritonX (x1), PBS-0.1% TritonX (x3), and PBS (x4). Urine samples were diluted in 100µl PBS-0.1% CETAB (Sigma, 29) at a total protein concentration of 0.055mg/ml, and following a 20 minute incubation in ice, were applied onto the arrays using a bioprocessor (Ciphergen Biosystems, Inc., Fremont, CA). After a 3hr-incubation at 4°C, the unbound urinary proteins were washed away by 5 washes with PBS-0.1 % CETAB (5min each, R.T.) followed by 5 washes with HPLC-H₂O, CHCA added, and the chip subjected to mass analysis. The spectra were generated using signal averaging of 90 laser shots.

A total of 3 positive and 3 negative urine specimens for this marker were analyzed. As shown in Figure 5A, marker UBC-1 was readily captured when the defensin-α Ab was pre-bound on the chip. In contrast, in the absence of the defensin Ab (Figure 5B) or in the presence of an unrelated Ab, no specific binding above the background levels was detected (Fig. 5C, 5D). Urine specimens that were UBC-1 negative by SELDI direct binding remained UBC-1 negative by the SELDI immunoassay (Fig. 5E).

Defensins form a family of small peptides with antimicrobial, cytotoxic and anti-tumor activities (Zhao *et al.,* FEBS Lett., 396:319-22 (1996)). Based on their primary structure, two families, the α and β defensins have been characterized in humans (Celis *et al., Electrophoresis,* 20(2):300-9 (1999)). β-defensins have been found to be primarily expressed in epithelial cells of the kidneys, skin, and respiratory system (Yang *et al., Science,* 286: 525-528 (1999); Selsted *et al., J Cell Biol.,* 118:929-936 (1992)) whereas α-defensins in neutrophils and intestinal Paneth cells (Mizukawa *et al., Anticancer Res.,* 19:2969-72 (1999)). Recent data further demonstrate the immunolocalization of α defensins in Langerhan cells and duct cells of submandibular glands of oral carcinoma patients (Mizukawa *et al., Anticancer Res.,* 20(2B):1125-7 (2000); Bamathan *et al., Amer. J. Pathol.,* 150 (3):1009-1019 (1997)) as well as endothelial and smooth muscle cells of coronary vessels (Porter *et al., FEBS Lett.,* 434:272-276 (1998)). The presence of defensin peptides in bladder cancer cells has not been reported before. Not wishing to be bound by a theory, this finding may be secondary to release of these peptides from tumor activated neutrophils; alternatively, these peptides may be expressed by the bladder cells, which can be confirmed by testing mRNA level in the bladder cells.

The presence of the Paneth cell-specific defensin in urine from ileal neobladder has been demonstrated (Hemmingsen *et al., J. Urol.,* 152:1923 (1994)), nevertheless, the presence of that type of defensin in urines from the same patient prior to cystectomy could not be shown. The antibody utilized in this example recognizes the neutrophil-specific defensins HNP1, 2, and 3, providing an explanation for the different results obtained in the two studies.

The presence of the defensin polypeptides in benign bladder cells suggests that, in contrast to urine, the presence of this marker is not tumor specific at the cellular level. However, not wishing to be bound by a theory, changes in its amount during tumorigenesis are expected to occur, resulting in the detection of higher levels in the urine from TCC patients. Alternatively, the presence of these polypeptides may also be indicative of the initial phases of tumorigenesis, not yet detected by the pathologist In support of this hypothesis is the fact that one patient was found with TCC stage T1, grade II three months after the collection of the bladder barbotage. In any case, immunoassays to monitor quantitative changes of this peptide may provide additional useful information with regard to tumor development and progression.

### VI. ANALYSIS OF COMBINATION OF MARKERS

The SELDI technology provides the advantage of analyzing multiple markers simultaneously. Therefore, to maximize the diagnostic utility of the TCC-associated biomarkers, the individual proteins UBC1-UBC5 and 7 protein clusters were placed in various combinations to form a biomarker panel, and the urine spectra for all groups re-analyzed. A biomarker combination was classified as positive if any marker of the combination set was present in a sample, and negative if none of the markers were detected in the specimen. Sensitivity and specificity of multiple biomarker panels are shown in Table 7 below.

**TABLE 7**

| *Marker (kD)* | *Sensitivity %* | *Specificity-N %* | *Specificity-O%* | *Specificity-All %* | *PPV%* | *NPV%* |
|---|---|---|---|---|---|---|
| 3.9/9.5/100 | 83.3 | 70.6 | 63.3 | 67.2 | 54.3 | 89.6 |
| 3.3/44/85-92 | 83.3 | 70.6 | 63.3 | 67.2 | 54.3 | 89.6 |
| 3.3/9.5/85-92 | 86.7 | 70.6 | 60.0 | 65.6 | 54.2 | 91.3 |

Using these biomarker panels, the sensitivity for detecting TCC increased from 43.3-63.3%, using individual biomarkers (Table 3) to 83.3%-86.7%. (Table 7). However, as expected, there was a compromise in the overall specificity of the assay, from an average of 81 % for single markers to 67.2% using a combination of biomarkers (Table 7). There was a notable increase in the NPV of the assay to 89.6% versus an average of 79% for a single marker, and the PPV of 54.3% (Table 7) was similar to the average PPV of 58% for a single assay (Table 3).

The combination of the 3.3/3.4kDa (UBC-1), 9.5kDa (UBC-2) markers and the 85-92kDa cluster (PC-7) was identified as the best of the biomarker combinations in terms of assay sensitivity. Using this set, a sensitivity of 86.7% was obtained with a PPV of 91.3%, and a specificity and NPV of 65.6% and 54.2%, respectively (Table 7).

All three of the combination sets shown on table 7, were capable of detecting low grade and stage carcinomas with relatively high sensitivity. Sensitivity of the biomarker panels versus the stage and grade of tumor are shown in Table 8 below.

**TABLE 8**

| *Marker (kD)* | *Grade I, II* | *Grade III* | *Ta* | *T1, T2,* T3 |
|---|---|---|---|---|
| 3.3/9.5/100 | 77.7 | 85.7 | 78.6 | 78.6 |
| 3.3/44/85-92 | 66.7 | 90.5 | 71.4 | 92.8 |
| 3.3/9.5/85-92 | 77.7 | 90.5 | 78.6 | 92.8 |

As shown in Table 8, the 3.3/3.4, 44 and 85-92kDa combination set detects 66.7% of grade I and II and 71.4% of stage Ta carcinomas. The 3.3/3.4, 9.5, 100kDa, and 3.3/3.4, 9.5 and 85-92kDa combination sets provided a slightly superior sensitivity of 77.7% for grades I and II and 78.6% for Ta carcinomas. Most notable was that the detection rate of the SELDI urine assay was markedly superior to the 33.3% rate obtained by either voided urine or bladder washing cytology for these same patients. All combination biomarker panels, provided higher sensitivities (85.7%-90.5%) in detecting grade III carcinomas and with the exception of the 3.3/3.4, 9.5 and 100kDa set, stage T1-T3 tumors (92.8%).

As described above, the sensitivity of each individual marker (UBC1 - UBC5) or each of the 7 protein cluster markers PC-1 through PC-7 for detecting TCC was found to be relatively low. However, combining the individual markers and protein clusters increased the overall TCC detection rate and the rate for low grade and low stage carcinomas. Based on the results described in this example section, the SELDI combinatorial approach provided a detection rate of 77.7% for grade I and II carcinomas and a detection rate of 78.6% for stage Ta carcinomas compared to a detection rate of 20-30% by voided urine cytology *(see* Grossman and Dinney, *Urol. Oncology* 5:3-10 (1999)). These results suggest that the markers of the present invention can be used (*e.g.*, using SELDI proteomic approach) for detecting early TCC.

The combinatorial biomarker analysis approach increased the sensitivity, but decreased the specificity of the assay. This approach relies on simple conventional statistical methods. For processing a larger pool of samples and SELDI data, it may be desirable to use an artificial intelligence program, such as fuzzy logic, cluster analysis or neural network (ANN) to analyze data. ANNs previously developed to predict outcome in prostate (Qureshi *et al., J. Urol*., 163: 630-633 (2000)) or bladder cancers (Snow, *et al., J. Urol.,* 152:1923 (1994)) based on clinicopathological and molecular markers can be applied in embodiments of the invention.

The present invention provides novel materials and methods for aiding bladder cancer diagnosis using markers that are differentially present in samples of a bladder cancer patient and a normal subject who does not have bladder cancer.

## Claims

1. A method for aiding a diagnosis of transitional cell carcinoma of the bladder, the method comprising:
(a) detecting at least one protein marker in a sample, wherein the protein marker is selected from:
Marker UBC-1: human defensin alpha2 and 1:
3.353 kDa ± 105 Da, 3.432 kDa ± 122 Da, 3,470 kDa ± 32 Da;
Marker PC-1: 4.950-5,150 kDa;
Marker PC-2: 5.710-6.000 kDa;
Marker PC-3: 6.758-7.750 kDa;
Marker PC-4: 15.000-16.000 kDa;
Marker PC-5: 37.500-40.000 kDa;
Marker PC-6: 79.500-82.000 kDa; and
Marker PC-7: 85.000-92.000 kDa; and
(b) correlating the detection of the marker or markers with a probable diagnosis of transitional cell carcinoma of the bladder.

2. The method of claim 1, wherein the correlation takes into account the presence or absence of the marker or markers in the sample and the frequency of detection of the same marker or markers in a control.

3. The method of claim 2, wherein the correlation further takes into account the amount of the marker or markers in the sample compared to a control amount of the marker or markers.

4. The method of claim 1, wherein the method comprises detecting at least three of the markers.

5. The method of claim 1, wherein the method comprises detecting at least four of the markers.

6. The method of claim 1, wherein the method comprises detecting at least five of the markers.

7. The method of claim 1, wherein the presence of markers UBC-1 and PC-7 is detected.

8. The method of claim 1, wherein the sample is urine.

9. The method of claim 1, wherein the sample is a cell lysate from a bladder barbotage.

10. The method of claim 1, wherein gas phase ion spectrometry is used for detecting the marker or markers.

11. The method of claim 10, wherein the gas phase ion spectrometry is laser desorption/ionization mass spectrometry.

12. The method of claim 11, wherein laser desorption/ionization mass spectrometry comprises:
(a) providing a substrate comprising an adsorbent attached thereto;
(b) contacting the sample with the adsorbent; and
(c) desorbing and ionizing the marker or markers from the substrate and detecting the desorbed/ionized marker or markers with the mass spectrometer.

13. The method of claim 12, wherein the substrate is a probe adapted for use with the mass spectrometer.

14. The method of claim 12, wherein the substrate is suitable for being placed on a probe which is adapted for use with the mass spectrometer.

15. The method of claim 12, wherein the adsorbent is an antibody that specifically binds to the marker.

16. The method of claim 12, wherein the adsorbent is a cationic adsorbent.

17. The method of claim 12, wherein the adsorbent is a metal chelating adsorbent.

18. The method of claim 17, comprising detecting Marker UBC-1.

19. The method of claim 1, wherein an immunoassay is used for detecting the marker or markers.

20. The method of claim 11, the method further comprising:
(a) generating data on the sample with the mass spectrometer indicating intensity of signal for mass/charge ratios;
(b) transforming the data into computer-readable form; and
(c) operating a computer to execute an algorithm, wherein the algorithm determines closeness-of-fit between the computer-readable data and data indicating a diagnosis of transitional cell carcinoma of the bladder or a negative diagnosis.

21. The method of claim 20, wherein the algorithm comprises an artificial intelligence program.

22. The method of claim 21, wherein the artificial intelligence program is a fuzzy logic, cluster analysis or neural network.

23. A method for aiding a diagnosis of transitional cell carcinoma of the bladder, the method comprising:
(a) detecting at least one protein marker in a sample, wherein the protein marker is selected from:
Marker PC-1: 4.950-5.150 kDa;
Marker PC-2: 5.710-6.000 kDa;
Marker PC-3: 6.758-7.750 kDa;
Marker PC-4: 15.000-16.000 kDa;
Marker PC-5: 37.500-40.000 kDa;
Marker PC-6: 79.500-82.000 kDa; and
Marker PC-7: 85.000-92.000 kDa; and
(b) correlating the detection of the marker or markers with a probable diagnosis of transitional cell carcinoma of the bladder.

24. A method according to claim 23, further comprising
(c) detecting one or more further protein markers in said sample, said markers being selected from
Marker UBC-1: human defensin alpha2 and 1:
3.353 kDa ± 105 Da, 3.432 kDa ± 122 Da, 3.470 kDa ± 32 Da;
Marker UBC-2: 9.495 kDa ± 233 Da;
Marker UBC-3: 44.6 kDa ± 1.9 Da;
Marker UBC-4: 100.120 kDa ± 4.3 Da; and
Marker UBC-5: 1.33.190 kDa ± 3.9 Da; and
(d) correlating the detection of the marker or markers with a probable diagnosis of transitional cell carcinoma of the bladder.

25. A method for aiding a diagnosis of transitional cell carcinoma of the bladder, the method comprising
(a) detecting at least three protein markers in a sample, wherein the protein markers are:
Marker UBC-1: human defensin alpha2 and 1:
3.353 kDa ± 105 Da, 3.432 kDa ± 122 Da, 3.470 kDa ± 32 Da;
Marker UBC-2; 9.495 kDa ± 233 Da;
Marker PC-7: 85.000-92.000 kDa; and
(b) correlating the detection of the marker or markers with a probable diagnosis of transitional cell carcinoma of the bladder.

26. A method for aiding a diagnosis of transitional cell carcinoma of the bladder, the method comprising
(a) detecting at least three protein markers in a sample, wherein the protein markers are:
Marker UBC-1: human defensin alpha2 and 1:
3.353 kDa ± 105 Da, 3.432 kDa ± 122 Da, 3.470 kDa ± 32 Da;
Marker UBC-3: 44.6 kDa ± 1.9 Da;
Marker PC-7: 85.000-92.000 kDa; and
(b) correlating the detection of the marker or markers with a probable diagnosis of transitional cell carcinoma of the bladder.

27. A method for aiding a diagnosis of transitional cell carcinoma of the bladder, the method comprising
(a) detecting at least three protein markers in a sample, wherein the protein markers are:
Marker UBC-1: human defensin alpha2 and 1:
3.353 kDa ± 105 Da, 3.432 kDa ± 122 Da, 3.470 kDa ± 32 Da;
Marker UBC-2: 9.495 kDa ± 233 Da;
Marker UBC-4: 100.120 kDa ± 4.3 Da; and
(b) correlating the detection of the marker or markers with a probable diagnosis of transitional cell carcinoma of the bladder.

28. A method for aiding a diagnosis of transitional cell carcinoma of the bladder, the method comprising
(a) detecting the protein Marker PC-7: 85.000-92.000 kDa in a sample, and
(b) detecting one or more further protein markers in said sample, said markers being selected from
Marker UBC-1: 3.353 kDa ± 105 Da, 3.432 kDa ± 122 Da,
3.470 kDa ± 32 Da;
Marker UBC-2: 9.495 kDa ± 233 Da;
Marker UBC-3: 44.6 kDa ± 1.9 Da;
Marker UBC-4: 100.120 kDa ± 4.3 Da; and
Marker UBC-5: 133.190 kDa ± 3.9 Da; and
(c) correlating the detection of the marker or markers with a probable diagnosis of transitional cell carcinoma of the bladder.

## Patentansprüche

1. Verfahren zur Unterstützung einer Diagnose von Übergangszellkarzinom der Blase, wobei das Verfahren folgendes umfaßt:
(a) Erfassen wenigstens eines Proteinmarkers in einer Probe, wobei der Proteinmarker unter den folgenden ausgewählt ist:
Marker UBC-1: humanes Defensin alpha2 und 1:
3.353 kDa ± 105 Da, 3.432 kDa ± 122 Da, 3.470 kDa ± 32 Da,
Marker PC-1: 4.950-5.150 kDa,
Marker PC-2: 5.710-6.000 kDa,
Marker PC-3: 6.758-7.750 kDa,
Marker PC-4: 15.000-16.000 kDa,
Marker PC-5: 37.500-40.000 kDa,
Marker PC-6: 79.500-82.000 kDa und
Marker PC-7: 85.000-92.000 kDa, und
(b) Korrelieren der Erfassung des Markers oder der Marker mit einer wahrscheinlichen Diagnose von Übergangszellkarzinom der Blase.

2. Verfahren nach Anspruch 1, wobei die Korrelation das Vorhandensein oder Nichtvorhandensein des Markers oder der Marker in der Probe und die Häufigkeit der Erfassung desselben Markers oder derselben Marker in einer Kontrolle berücksichtigt.

3. Verfahren nach Anspruch 2, wobei die Korrelation weiterhin die Menge des Markers oder der Marker in der Probe im Vergleich zu einer Kontrollmenge des Markers oder der Marker berücksichtigt.

4. Verfahren nach Anspruch 1, wobei das Verfahren das Erfassen von wenigstens dreien der Marker umfaßt.

5. Verfahren nach Anspruch 1, wobei das Verfahren das Erfassen von wenigstens vieren der Marker umfaßt.

6. Verfahren nach Anspruch 1, wobei das Verfahren das Erfassen von wenigstens fünfen der Marker umfaßt.

7. Verfahren nach Anspruch 1, wobei das Vorliegen der Marker UBC-1 und PC-7 erfaßt wird.

8. Verfahren nach Anspruch 1, wobei die Probe Urin ist.

9. Verfahren nach Anspruch 1, wobei die Probe ein Zelllysat von einer Blasenbarbotage ist.

10. Verfahren nach Anspruch 1, wobei Gasphasen-lonenspektrometrie für die Erfassung des Markers oder der Marker verwendet wird.

11. Verfahren nach Anspruch 10, wobei die Gasphasen-lonenspektrometrie Laserdesorptions/lonisations-Massenspektrometrie ist.

12. Verfahren nach Anspruch 11, wobei die Laserdesorptions/lonisations-Massenspektrometrie folgendes umfaßt:
(a) Bereitstellen eines Substrats, welches ein daran angebrachtes Adsorptionsmittel umfaßt,
(b) Inkontaktbringen der Probe mit dem Adsorptionsmittel und
(c) Desorbieren und Ionisieren des Markers oder der Marker von dem Substrat und Erfassen des desorbierten/ionisierten Markers oder der Marker mit dem Massenspektrometer.

13. Verfahren nach Anspruch 12, wobei das Substrat eine Sonde ist, die für eine Verwendung mit dem Massenspektrometer ausgestaltet ist.

14. Verfahren nach Anspruch 12, wobei das Substrat dafür geeignet ist, auf einer Sonde plaziert zu werden, die für eine Verwendung mit dem Massenspektrometer ausgestaltet ist.

15. Verfahren nach Anspruch 12, wobei das Adsorptionsmittel ein Antikörper ist, der spezifisch an den Marker bindet.

16. Verfahren nach Anspruch 12, wobei das Adsorptionsmittel ein kationisches Adsorptionsmittel ist.

17. Verfahren nach Anspruch 12, wobei das Adsorptionsmittel ein Metall chelierendes Adsorptionsmittel ist.

18. Verfahren nach Anspruch 17, welches das Erfassen des Markers UBC-1 umfaßt.

19. Verfahren nach Anspruch 1, wobei ein Immunoassay verwendet wird, um den Marker oder die Marker zu erfassen.

20. Verfahren nach Anspruch 11, wobei das Verfahren weiterhin folgendes umfaßt:
(a) Erzeugen von Daten über die Probe mit dem Massenspektrometer, die die Intensität des Signals für Masse/Beladungsverhältnisse anzeigen,
(b) Umwandeln der Daten in computerlesbare Form und
(c) Betreiben eines Computers, um einen Algorithmus auszuführen, wobei der Algorithmus das Ausmaß der Übereinstimmung zwischen den computerlesbaren Daten und den Daten, die eine Diagnose von Übergangszellkarzinom der Blase oder eine negative Diagnose anzeigen, bestimmt.

21. Verfahren nach Anspruch 20, wobei der Algorithmus ein Programm künstlicher Intelligenz umfaßt.

22. Verfahren nach Anspruch 21, wobei das Programm künstlicher Intelligenz eine Fuzzy-Logik, eine Clusteranalyse oder ein neuronales Netz ist.

23. Verfahren zur Unterstützung einer Diagnose von Übergangszellkarzinom der Blase, wobei das Verfahren folgendes umfaßt:
(a) Erfassen wenigstens eines Proteinmarkers in einer Probe, wobei der Proteinmarker unter den folgenden ausgewählt ist:
Marker PC-1: 4.950-5.150 kDa,
Marker PC-2: 5.710-6.000 kDa,
Marker PC-3: 6.758-7.750 kDa,
Marker PC-4: 15.000-16.000 kDa,
Marker PC-5: 37.500-40.000 kDa,
Marker PC-6: 79.500-82.000 kDa und
Marker PC-7: 85.000-92.000 kDa, und
(b) Korrelieren der Erfassung des Markers oder der Marker mit einer wahrscheinlichen Diagnose von Übergangszellkarzinom der Blase.

24. Verfahren nach Anspruch 23, welches weiterhin folgendes umfaßt:
(c) Erfassen eines oder mehrerer weiterer Proteinmarker in der Probe, wobei die Marker unter folgenden ausgewählt sind:
Marker UBC-1: humanes Defensin alpha2 und 1:
3.353 kDa ± 105 Da, 3.432 kDa ± 122 Da, 3.470 kDa ± 32 Da,
Marker UBC-2: 9.495 kDa ± 233 Da,
Marker UBC-3: 44,6 kDa ± 1,9 Da,
Marker UBC-4: 100.120 kDa ± 4,3 Da und
Marker UBC-5: 133.190 kDa ± 3,9 Da, und
(d) Korrelieren der Erfassung des Markers oder der Marker mit einer wahrscheinlichen Diagnose von Übergangszellkarzinom der Blase.

25. Verfahren zur Unterstützung einer Diagnose von Übergangszellkarzinom der Blase, wobei das Verfahren folgendes umfaßt:
(a) Erfassen wenigstens dreier Proteinmarker in einer Probe, wobei die Proteinmarker die folgenden sind:
Marker UBC-1: humanes Defensin alpha2 und 1:
3.353 kDa ± 105 Da, 3.432 kDa ± 122 Da, 3.470 kDa ± 32 Da,
Marker UBC-2: 9.495 kDa ± 233 Da,
Marker PC-7: 85.000-92.000 kDa, und
(b) Korrelieren der Erfassung des Markers oder der Marker mit einer wahrscheinlichen Diagnose von Übergangszellkarzinom der Blase.

26. Verfahren zur Unterstützung einer Diagnose von Übergangszellkarzinom der Blase, wobei das Verfahren folgendes umfaßt:
(a) Erfassen wenigstens dreier Proteinmarker in einer Probe, wobei die Proteinmarker die folgenden sind:
Marker UBC-1: humanes Defensin alpha2 und 1:
3.353 kDa ± 105 Da, 3.432 kDa ± 122 Da, 3.470 kDa ± 32 Da,
Marker UBC-3: 44,6 kDa ± 1,9 Da,
Marker PC-7: 85.000-92.000 kDa, und
(b) Korrelieren der Erfassung des Markers oder der Marker mit einer wahrscheinlichen Diagnose von Übergangszellkarzinom der Blase.

27. Verfahren zur Unterstützung einer Diagnose von Übergangszellkarzinom der Blase, wobei das Verfahren folgendes umfaßt:
(a) Erfassen wenigstens dreier Proteinmarker in einer Probe, wobei die Proteinmarker die folgenden sind:
Marker UBC-1: humanes Defensin alpha2 und 1:
3.353 kDa ± 105 Da, 3.432 kDa ± 122 Da, 3.470 kDa ± 32 Da,
Marker UBC-2: 9.495 kDa ± 233 Da,
Marker UBC-4: 100.120 kDa ± 4.3 Da, und
(b) Korrelieren der Erfassung des Markers oder der Marker mit einer wahrscheinlichen Diagnose von Übergangszellkarzinom der Blase.

28. Verfahren zur Unterstützung einer Diagnose von Übergangszellkarzinom der Blase, wobei das Verfahren folgendes umfaßt:
(a) Erfassen des Proteinmarkers PC-7: 85.000-92.000 kDa in einer Probe und
(b) Erfassen eines oder mehrerer weiterer Proteinmarker in der Probe, wobei die Marker unter den folgenden ausgewählt sind:
Marker UBC-1: 3.353 kDa ± 105 Da, 3.432 kDa ± 122 Da, 3.470 kDa ± 32 Da,
Marker UBC-2: 9.495 kDa ± 233 Da,
Marker UBC-3: 44,6 kDa ± 1,9 Da,
Marker UBC-4: 100.120 kDa ± 4,3 Da und
Marker UBC-5: 133.190 kDa ± 3,9 Da, und
(c) Korrelieren der Erfassung des Markers oder der Marker mit einer wahrscheinlichen Diagnose von Übergangszellkarzinom der Blase.

## Revendications

1. Procédé d'aide au diagnostic d'un carcinome cellulaire de type transitionnel de la vessie, ledit procédé comprenant :
(a) la détection d'au moins un marqueur protéique dans un échantillon, ledit marqueur protéique étant choisi parmi :
le Marqueur UBC-1 : défensines alpha-1 et alpha-2 humaines :
3,353 kDa ± 105 Da, 3,432 kDa ± 122 Da, 3,470 kDa + 32 Da ;
le Marqueur PC-1 : 4,950 - 5,150 kDa ;
le Marqueur PC-2 : 5,710 - 6,000 kDa ;
le Marqueur PC-3 : 6,758 - 7,750 kDa ;
le Marqueur PC-4 : 15,000 - 16,000 kDa ;
le Marqueur PC-5 : 37,500 - 40,000 kDa ;
le Marqueur PC-6 : 79,500 - 82,000 kDa ; et
le Marqueur PC-7 : 85,000 - 92,000 kDa ; et
(b) la corrélation de la détection du ou des marqueur(s) avec un diagnostic probable de carcinome cellulaire de type transitionnel de la vessie.

2. Procédé selon la revendication 1, dans lequel la corrélation prend en compte la présence ou l'absence du ou des marqueur(s) dans l'échantillon et la fréquence de détection du ou des même(s) marqueur(s) dans un témoin.

3. Procédé selon la revendication 2, dans lequel la corrélation prend en compte, en outre, la quantité du ou des marqueur(s) dans l'échantillon comparativement à une quantité témoin du ou des marqueur(s).

4. Procédé selon la revendication 1, ledit procédé comprenant la détection d'au moins trois des marqueurs.

5. Procédé selon la revendication 1, ledit procédé comprenant la détection d'au moins quatre des marqueurs.

6. Procédé selon la revendication 1, ledit procédé comprenant la détection d'au moins cinq des marqueurs.

7. Procédé selon la revendication 1, dans lequel la présence des marqueurs UBC-1 et PC-7 est détectée.

8. Procédé selon la revendication 1, dans lequel l'échantillon est de l'urine.

9. Procédé selon la revendication 1, dans lequel l'échantillon est un lysat cellulaire provenant d'un barbotage de la vessie.

10. Procédé selon la revendication 1, dans lequel on utilise la spectrométrie ionique en phase gazeuse pour détecter le ou les marqueur(s).

11. Procédé selon la revendication 10, dans lequel la spectrométrie ionique en phase gazeuse est la spectrométrie de masse avec désorption/ionisation laser.

12. Procédé selon la revendication 11, dans lequel la spectrométrie de masse avec désorption/ionisation laser comprend :
(a) la fourniture d'un substrat comprenant un adsorbant qui lui est attaché ;
(b) la mise en contact de l'échantillon avec l'adsorbant ; et
(c) la désorption et l'ionisation du ou des marqueur(s) à partir du substrat
et la détection du ou des marqueur(s) désorbé(s)/ionisé(s) avec le spectromètre de masse.

13. Procédé selon la revendication 12, dans lequel le substrat est une sonde adaptée pour être utilisée avec le spectromètre de masse.

14. Procédé selon la revendication 12, dans lequel le substrat est approprié pour être placé sur une sonde qui est adaptée pour être utilisée avec le spectromètre de masse.

15. Procédé selon la revendication 12, dans lequel l'adsorbant est un anticorps qui se lie spécifiquement au marqueur.

16. Procédé selon la revendication 12, dans lequel l'adsorbant est un adsorbant cationique.

17. Procédé selon la revendication 12, dans lequel l'adsorbant est un adsorbant chélatant un métal.

18. Procédé selon la revendication 17, comprenant la détection du Marqueur UBC-1.

19. Procédé selon la revendication 1, dans lequel on utilise un immunodosage pour détecter le ou les marqueur(s).

20. Procédé selon la revendication 11, ledit procédé comprenant en outre :
(a) la production de données sur l'échantillon avec le spectromètre de masse indiquant l'intensité du signal pour des rapports de masse/charge ;
(b) la transformation des données dans une forme informatique ; et
(c) la mise en oeuvre d'un ordinateur pour exécuter un algorithme, l'algorithme déterminant le degré d'accord entre les données informatiques et les données indiquant un diagnostic de carcinome cellulaire de type transitionnel de la vessie ou un diagnostic négatif.

21. Procédé selon la revendication 20, dans lequel l'algorithme comprend un programme d'intelligence artificielle.

22. Procédé selon la revendication 21, dans lequel le programme d'intelligence artificielle est une logique floue, une analyse par amas ou un réseau neuronal.

23. Procédé d'aide au diagnostic d'un carcinome de type transitionnel de la vessie, ledit procédé comprenant :
(a) la détection d'au moins un marqueur protéique dans un échantillon, ledit marqueur protéique étant choisi parmi :
le Marqueur PC-1 : 4,950 - 5,150 kDa ;
le Marqueur PC-2 : 5,710 - 6,000 kDa ;
le Marqueur PC-3 : 6,758 - 7,750 kDa ;
le Marqueur PC-4 : 15,000 - 16,000 kDa ;
le Marqueur PC-5 : 37,500 - 40,000 kDa ;
le Marqueur PC-6 : 79,500 - 82,000 kDa ; et
le Marqueur PC-7 : 85,000 - 92,000 kDa ; et
(b) la corrélation de la détection du ou des marqueur(s) avec un diagnostic probable de carcinome cellulaire de type transitionnel de la vessie.

24. Procédé selon la revendication 23, comprenant en outre :
(c) la détection d'un ou de plusieurs autres marqueurs protéiques dans ledit échantillon, lesdits marqueurs étant choisis parmi :
le Marqueur UBC-1 : défensines alpha-1 et alpha-2 humaines :
3,353 kDa ± 105 Da, 3,432 kDa ± 122 Da, 3,470 kDa ± 32 Da ;
le Marqueur UBC-2 : 9,495 kDa ± 233 Da ;
le Marqueur UBC-3 : 44,6 kDa ± 1,9 Da ;
le Marqueur UBC-4 : 100,120 kDa ± 4,3 Da ; et
le Marqueur UBC-5 : 133,190 kDa ± 3,9 Da ; et
(d) la corrélation de la détection du ou des marqueur(s) avec un diagnostic probable de carcinome cellulaire de type transitionnel de la vessie.

25. Procédé d'aide au diagnostic d'un carcinome cellulaire de type transitionnel de la vessie, ledit procédé comprenant :
(a) la détection d'au moins trois marqueurs protéiques dans un échantillon, lesdits marqueurs protéiques étant :
le Marqueur UBC-1 : défensines alpha-1 et alpha-2 humaines :
3,353 kDa ± 105 Da, 3,432 kDa ± 122 Da, 3,470 kDa ± 32 Da ;
le Marqueur UBC-2 : 9,495 kDa ± 233 Da ;
le Marqueur PC-7 : 85,000 - 92,000 kDa ; et
(b) la corrélation de la détection du ou des marqueur(s) avec un diagnostic probable de carcinome cellulaire de type transitionnel de la vessie.

26. Procédé d'aide au diagnostic d'un carcinome cellulaire de type transitionnel de la vessie, ledit procédé comprenant :
(a) la détection d'au moins trois marqueurs protéiques dans un échantillon, lesdits marqueurs protéiques étant :
le Marqueur UBC-1 : défensines alpha-1 et alpha-2 humaines :
3,353 kDa ± 105 Da, 3,432 kDa ± 122 Da, 3,470 kDa ± 32 Da ;
le Marqueur UBC-3 : 44,6 kDa ± 1,9 Da ;
le Marqueur PC-7 : 85,000 - 92,000 kDa ; et
(b) la corrélation de la détection du ou des marqueur(s) avec un diagnostic probable de carcinome cellulaire de type transitionnel de la vessie.

27. Procédé d'aide au diagnostic d'un carcinome cellulaire de type transitionnel de la vessie, ledit procédé comprenant :
(a) la détection d'au moins trois marqueurs protéiques dans un échantillon, lesdits marqueurs protéiques étant :
le Marqueur UBC-1 : défensines alpha-1 et alpha-2 humaines :
3,353 kDa ± 105 Da, 3,432 kDa ± 122 Da, 3,470 kDa ± 32 Da ;
le Marqueur UBC-2 : 9,495 kDa ± 233 Da ;
le Marqueur UBC-4 : 100,120 kDa ± 4,3 Da ; et
(b) la corrélation de la détection du ou des marqueur(s) avec un diagnostic probable de carcinome cellulaire de type transitionnel de la vessie.

28. Procédé d'aide au diagnostic d'un carcinome cellulaire de type transitionnel de la vessie, ledit procédé comprenant :
la détection du Marqueur protéique PC-7 : 85,000 - 92,000 kDa dans un échantillon, et
la détection d'un ou de plusieurs autre(s) marqueur(s) protéique(s) dans ledit échantillon, lesdits marqueurs étant choisis parmi :
le Marqueur UBC-1 : 3,353 kDa ± 105 Da, 3,432 kDa ± 122 Da, 3,470 kDa ± 32 Da ;
le Marqueur UBC-2 : 9,495 kDa ± 233 Da ;
le Marqueur UBC-3 : 44,6 kDa ± 1,9 Da ;
le Marqueur UBC-4 : 100,120 kDa ± 4,3 Da ; et
le Marqueur UBC-5 : 133,190 kDa ± 3,9 Da ; et
la corrélation de la détection du ou des marqueur(s) avec un diagnostic probable de carcinome cellulaire de type transitionnel de la vessie.
